# EUROPEAN PATENT APPLICATION

(11) **EP 4 700 027 A1**
(43) Date of publication of application: **25.02.2026**
(21) Application number: 24792130.7
(22) Date of filing: 19.04.2024
(51) Int. Cl.: C07D 487/16, C07D 487/12, A61K 31/4985, A61P 35/00

(54) **CRYSTALLINE FORM OF NITROGEN-CONTAINING TETRACYCLIC COMPOUND AND PREPARATION METHOD THEREFOR**

(30) Priority: 21.04.2023 CN 202310433998
(71) Applicant: Jiangsu Hengrui Pharmaceuticals Co., Ltd., Lianyungang, Jiangsu 222047 (CN); Shandong Suncadia Medicine Co., Ltd., Jinan, Shandong 250104 (CN)
(72) Inventor: LIU, Dongqing, Jinan, Shandong 250104 (CN); WU, Qi, Lianyungang, Jiangsu 222047 (CN); LU, Yu, Jinan, Shandong 250104 (CN); DU, Zhenxing, Lianyungang, Jiangsu 222047 (CN); ZHU, Ning, Jinan, Shandong 250104 (CN); ZHAO, Xianghu, Lianyungang, Jiangsu 222047 (CN)
(74) Representative: Potter Clarkson
(86) International application number: PCT/CN2024/088832
(87) International publication number: WO 2024/217545

(57) **Abstract**

The present disclosure relates to a crystalline form of a nitrogen-containing tetracyclic compound and a preparation method therefor. Specifically, provided in the present disclosure are a crystal form C, a crystal form F, a crystal form G, a crystal form H, a crystal form L, a crystal form M, a crystal form N, a crystal form R and a crystal form S of (*S*)-4-((*S*)-10-acryloyl-4-chloro-2-fluoro-14-oxo-8,8*a*,9,10,11,12-hexahydro-7*H*,14*H*-pyrazino[1',2':5,6][1,5]diazocin[3,2,1-*hi*]indazol-3-yl)-2-amino-7-fluorobenzo[*b*]thiophene-3-carbonitrile (formula 1), which crystal forms have good stability and can be better used in clinical treatment.

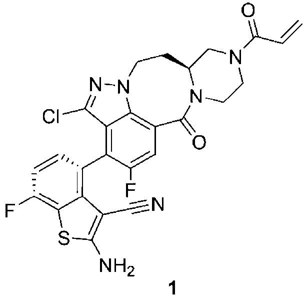

## Description

### TECHNICAL FIELD

The present disclosure pertains to the technical field of pharmaceutics and relates to a crystal form of a nitrogen-containing tetracyclic compound and a method for preparing same.

### BACKGROUND

The RAS (rat sarcoma viral oncogene homolog) family belongs to the small GTPase superfamily and is widely expressed in various eukaryotes. There are three RAS genes (HRAS, KRAS, and NRAS) in humans, which are expressed as four highly related RAS small GTPases (HRAS, KRAS4A, KRAS4B, and NRAS). They act as binary switches for GDP-GTP regulation. They generally exist in two forms: a GDP (guanosine diphosphate)-bound form when in an inactive state and a GTP (guanosine triphosphate)-bound form when in an activated state. RAS proteins regulate multiple downstream pathways including RAF-MEK-ERK and PI3K/Akt/mTOR by switching between the two active states, thereby affecting cell growth, proliferation, and differentiation (Nat Rev Cancer, 2007, 7, 295-308). The RAS genes exhibit high mutation rates in various tumors such as pancreatic cancer, colorectal cancer, and non-small cell lung cancer. Activated mutant RAS proteins promote abnormal signal transduction, thereby causing cancer development and progression, as well as resistance to targeted drugs. The KRAS mutation is the gene with the highest mutation rate among human oncogenes and accounts for 20%-30% of all tumors.

KRAS G12C has drawn numerous renowned pharmaceutical companies around the world to the research and development of related new drugs. Although the fastest progressing small-molecule KRAS G12C inhibitor, Sotorasib (AMG510) by Amgen, was approved by the FDA on May 28, 2021, for patients with non-small cell lung cancer carrying the KRAS G12C mutation who have previously undergone at least one systemic treatment, Eli Lilly and Company's next-generation KRAS G12C inhibitor LY3537982 has been attracting even more interest. Eli Lilly and Company presented preclinical data on LY3537982 at the American Association for Cancer Research (AACR) Annual Meeting in April 2021. The data revealed that LY3537982 is over 10 times more effective than Sotorasib in inhibiting cellular activity, and it entered Phase I clinical trials in July 2021. Thus, there is still a clinical need for KRAS G12C inhibitors that are highly selective, safe, and effective.

PCT/CN2022/126650 provides a KRAS G12C inhibitor chemically named (*S*)-4-((*S*)-10-acryloyl-4-chloro-2-fluoro-14-oxo-8,8a,9,10,11,12-hexahydro-7*H*,14*H*-pyrazino[1',2':5,6][1,5]diazocino[3,2,1-*hi*]indazol-3-yl)-2-amino-7-fluorobenzo[*b*]thiophene-3-carbonitrile, having a structure represented by formula 1:

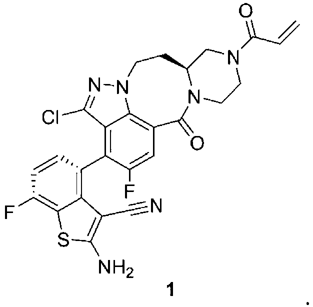

A crystal form used as an active pharmaceutical ingredient often influences the chemical stability of the drug. Variations in crystallization conditions or storage conditions may cause changes in the crystal form structure of the compound and sometimes the generation of other crystal forms. Generally, amorphous drug products have no regular crystal form structure and often have other defects, such as poor product stability, fine particles, difficult filtration, proneness to agglomeration, and poor flowability. Polymorphs of a drug impose different requirements on product storage, production, and scale-up. Therefore, it is necessary to extensively study the crystal forms of the aforementioned compound and to improve various properties of the aforementioned compound.

### SUMMARY

The present disclosure provides a new crystal form of the compound represented by formula 1, which has good stability and better clinical prospects,

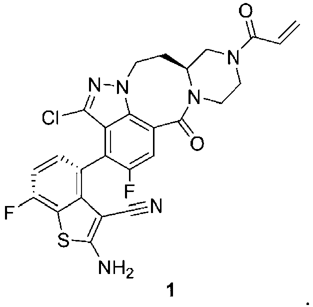

The present disclosure provides a crystal form A of the compound represented by formula 1, wherein an X-ray powder diffraction pattern expressed in terms of 2*θ* angles, which are diffraction angles, of the crystal form has characteristic peaks at 8.406, 10.960, 11.530, 18.216, and 22.849.

In some embodiments, the X-ray powder diffraction pattern expressed in terms of 2*θ* angles, which are diffraction angles, of the crystal form A of the compound represented by formula 1 has characteristic peaks at 7.419, 8.406, 10.960, 11.530, 18.216, 21.298, 22.849, and 28.948.

In some embodiments, the X-ray powder diffraction pattern expressed in terms of 2*θ* angles, which are diffraction angles, of the crystal form A of the compound represented by formula 1 has characteristic peaks at 7.419, 8.406, 10.960, 11.530, 18.216, 21.298, 22.433, 22.849, 28.948, and 30.452.

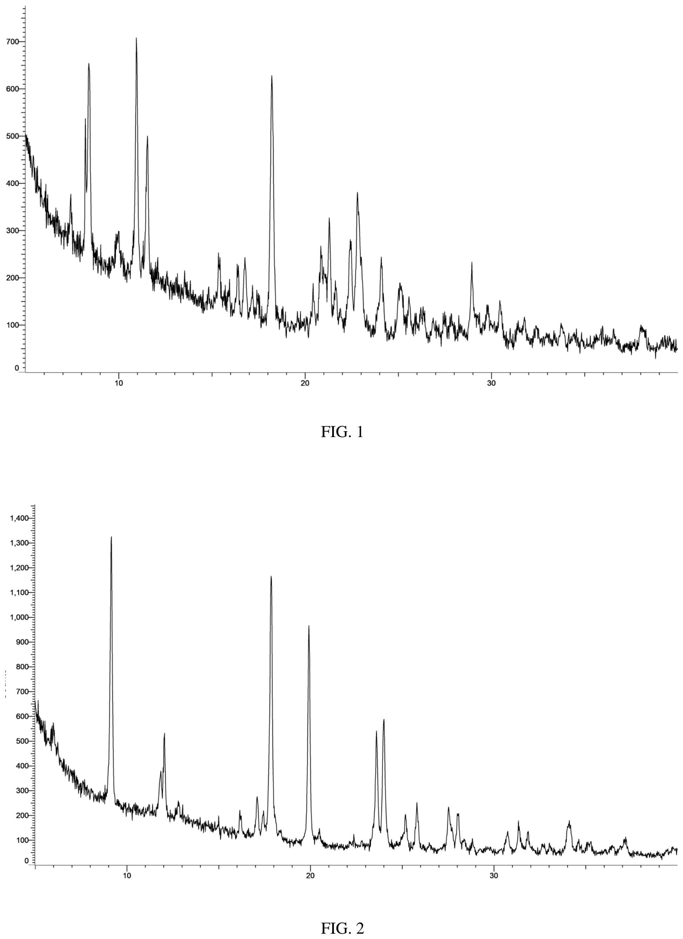

In some embodiments, the X-ray powder diffraction pattern expressed in terms of 2*θ* angles, which are diffraction angles, of the crystal form A of the compound represented by formula 1 is shown in FIG. 1.

The present disclosure further provides a method for preparing the crystal form A of the compound represented by formula 1, the method comprising adding the compound of formula 1 to a solvent a and stirring for crystallization, wherein the solvent a is selected from the group consisting of one or more of ethyl acetate and n-heptane.

The present disclosure provides a crystal form C of the compound represented by formula 1, wherein an X-ray powder diffraction pattern expressed in terms of 2*θ* angles, which are diffraction angles, of the crystal form has characteristic peaks at 9.150, 12.034, 17.860, 19.920, 23.604, and 23.997.

In some embodiments, the X-ray powder diffraction pattern expressed in terms of 2*θ* angles, which are diffraction angles, of the crystal form C of the compound represented by formula 1 has characteristic peaks at 9.150, 11.840, 12.034, 17.860, 19.920, 23.604, 23.997, 25.803, 27.536, and 28.038.

In some embodiments, the X-ray powder diffraction pattern expressed in terms of 2*θ* angles, which are diffraction angles, of the crystal form C of the compound represented by formula 1 has characteristic peaks at 9.150, 11.840, 12.034, 17.860, 19.920, 23.604, 23.997, 25.803, 27.536, 28.038, 30.743, and 31.353.

In some embodiments, the X-ray powder diffraction pattern expressed in terms of 2*θ* angles, which are diffraction angles, of the crystal form C of the compound represented by formula 1 is shown in FIG. 2.

The present disclosure further provides a method for preparing the crystal form C of the compound represented by formula 1, the method comprising:
method I: adding the compound of formula 1 to a solvent I, and stirring for crystallization, wherein the solvent I is selected from the group consisting of one or more of isopropanol, ethanol, and n-propanol;
method II: dissolving the compound of formula 1 in 1,4-dioxane, adding a solvent II, and stirring for crystallization, wherein the solvent II is selected from the group consisting of one or more of isopropanol, methyl *tert*-butyl ether, *n*-heptane, isopropyl acetate, dichloromethane, and cyclohexane;
method III: dissolving the compound of formula 1 in a solvent III, and stirring for crystallization, wherein the solvent III is selected from the group consisting of one or more of acetone, tetrahydrofuran, 1,4-dioxane, isopropyl acetate, dichloromethane, and tetrahydrofuran/ethanol (v/v = 2:1).

The present disclosure provides a crystal form D of the compound represented by formula 1, wherein an X-ray powder diffraction pattern expressed in terms of 2*θ* angles, which are diffraction angles, of the crystal form has characteristic peaks at 9.096, 11.735, 12.094, 17.928, and 23.968.

In some embodiments, the X-ray powder diffraction pattern expressed in terms of 2*θ* angles, which are diffraction angles, of the crystal form D of the compound represented by formula 1 has characteristic peaks at 9.096, 11.735, 12.094, 14.894, 16.002, 17.928, 19.870, 23.397, and 23.968.

In some embodiments, the X-ray powder diffraction pattern expressed in terms of 2*θ* angles, which are diffraction angles, of the crystal form D of the compound represented by formula 1 has characteristic peaks at 9.096, 11.735, 12.094, 14.894, 16.002, 17.928, 19.870, 23.397, 23.968, 24.917, and 29.556.

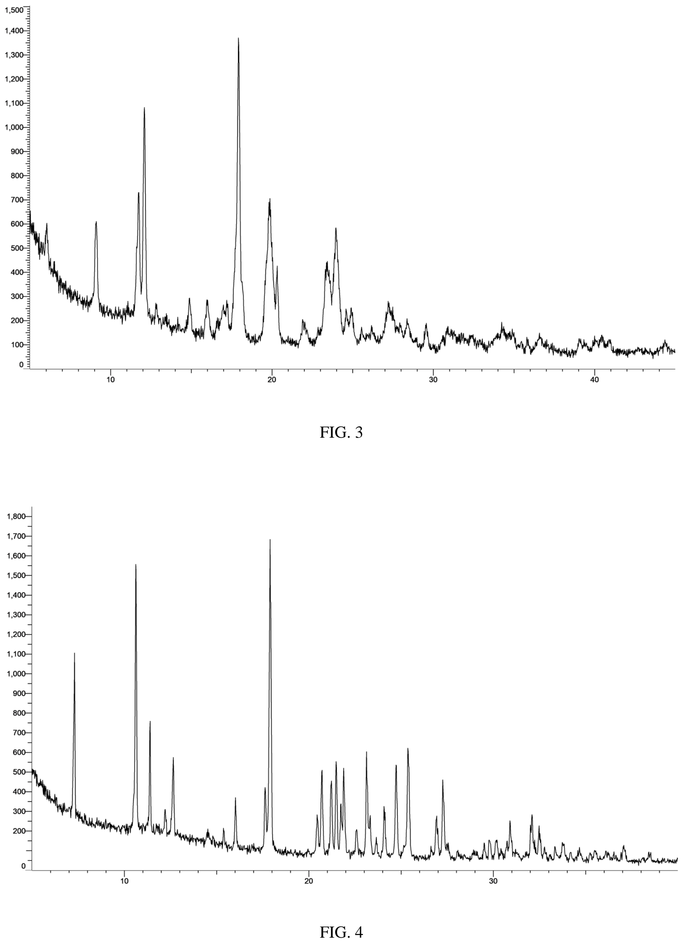

In some embodiments, the X-ray powder diffraction pattern expressed in terms of 2*θ* angles, which are diffraction angles, of the crystal form D of the compound represented by formula 1 is shown in FIG. 3.

The present disclosure further provides a method for preparing the crystal form D of the compound represented by formula 1, the method comprising adding the compound of formula 1 to a solvent b and stirring for crystallization, wherein the solvent b is selected from the group consisting of one or more of *n*-butanol and *tert*-butanol.

The present disclosure provides a crystal form E of the compound represented by formula 1, wherein an X-ray powder diffraction pattern expressed in terms of 2*θ* angles, which are diffraction angles, of the crystal form has characteristic peaks at 7.296, 10.626, 17.905, 23.131, and 25.377.

In some embodiments, the X-ray powder diffraction pattern expressed in terms of 2*θ* angles, which are diffraction angles, of the crystal form E of the compound represented by formula 1 has characteristic peaks at 7.296, 10.626, 11.393, 12.647, 17.905, 20.706, 23.131, and 25.377.

In some embodiments, the X-ray powder diffraction pattern expressed in terms of 2*θ* angles, which are diffraction angles, of the crystal form E of the compound represented by formula 1 has characteristic peaks at 7.296, 10.626, 11.393, 12.647, 16.030, 17.905, 20.706, 21.483, 23.131, 24.729, 25.377, and 27.267.

In some embodiments, the X-ray powder diffraction pattern expressed in terms of 2*θ* angles, which are diffraction angles, of the crystal form E of the compound represented by formula 1 is shown in FIG. 4.

The present disclosure further provides a method for preparing the crystal form E of the compound represented by formula 1, the method comprising:
method I: dissolving the compound of formula 1 in methanol, and volatilizing the solvent;
method II: adding the compound of formula 1 to 10% water/methanol, and stirring.

The present disclosure provides a crystal form F of the compound represented by formula 1, wherein an X-ray powder diffraction pattern expressed in terms of 2*θ* angles, which are diffraction angles, of the crystal form has characteristic peaks at 9.947, 15.672, 18.740, 20.712, 23.910, and 28.351.

In some embodiments, the X-ray powder diffraction pattern expressed in terms of 2*θ* angles, which are diffraction angles, of the crystal form F of the compound represented by formula 1 has characteristic peaks at 7.935, 9.947, 10.339, 15.672, 18.740, 19.933, 20.712, 23.910, 25.504, 26.139, and 28.351.

In some embodiments, the X-ray powder diffraction pattern expressed in terms of 2*θ* angles, which are diffraction angles, of the crystal form F of the compound represented by formula 1 has characteristic peaks at 7.935, 9.947, 10.339, 11.651, 11.990, 15.672, 16.642, 18.740, 19.933, 20.712, 21.471, 23.910, 25.504, 26.139, 27.660, 28.351, and 28.898.

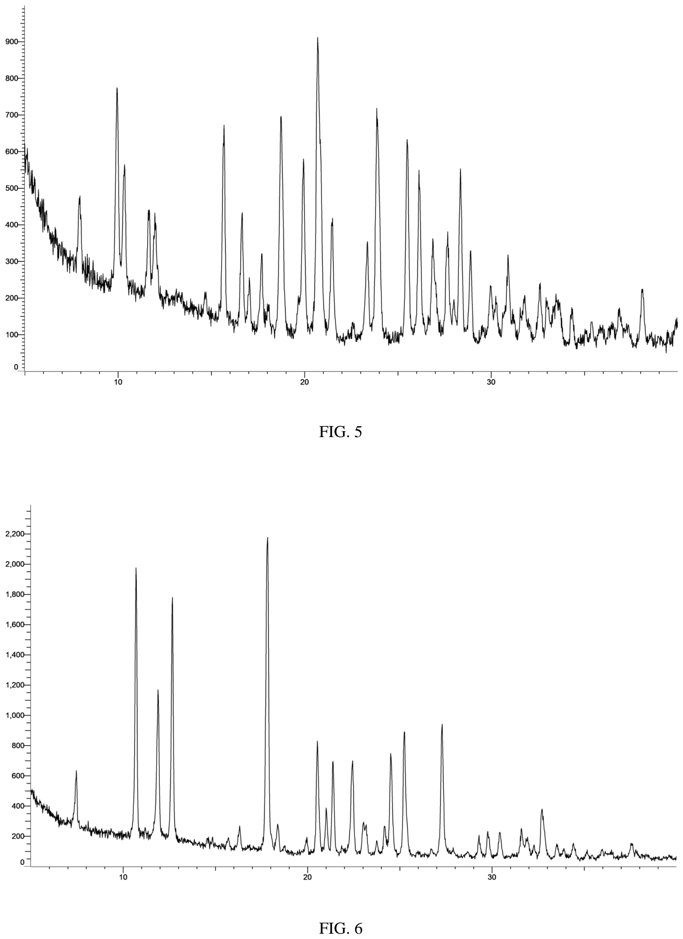

In some embodiments, the X-ray powder diffraction pattern expressed in terms of 2*θ* angles, which are diffraction angles, of the crystal form F of the compound represented by formula 1 is shown in FIG. 5.

The present disclosure further provides a method for preparing the crystal form F of the compound represented by formula 1, the method comprising:
method I: adding the compound of formula 1 to purified water, and cyclically heating and cooling between 50 °C and 5 °C with stirring;
method II: dissolving the compound of formula 1 in 80% acetone/water, adding purified water, and stirring.

In the present disclosure, heating and cooling between 50 °C and 5 °C refers to an operation of repeatedly cycling by raising the temperature from 5 °C to 50 °C and then lowering the temperature from 50 °C to 5 °C. In some embodiments, the temperature is lowered from 50 °C to 5 °C within 1 h and then raised from 5 °C to 50 °C within 1 h.

The present disclosure provides a crystal form G of the compound represented by formula 1, wherein an X-ray powder diffraction pattern expressed in terms of 2*θ* angles, which are diffraction angles, of the crystal form has characteristic peaks at 10.701, 11.896, 12.669, 17.822, 25.246, and 27.288.

In some embodiments, the X-ray powder diffraction pattern expressed in terms of 2*θ* angles, which are diffraction angles, of the crystal form G of the compound represented by formula 1 has characteristic peaks at 7.457, 10.701, 11.896, 12.669, 17.822, 20.532, 21.378, 25.246, 27.288, and 32.704.

In some embodiments, the X-ray powder diffraction pattern expressed in terms of 2*θ* angles, which are diffraction angles, of the crystal form G of the compound represented by formula 1 has characteristic peaks at 7.457, 10.701, 11.896, 12.669, 17.822, 20.532, 21.015, 21.378, 22.429, 24.516, 25.246, 27.288, and 32.704.

In some embodiments, the X-ray powder diffraction pattern expressed in terms of 2*θ* angles, which are diffraction angles, of the crystal form G of the compound represented by formula 1 is shown in FIG. 6.

The present disclosure further provides a method for preparing the crystal form G of the compound represented by formula 1, the method comprising:
method I: adding the compound of formula 1 to methanol, and cyclically heating and cooling between 50 °C and 5 °C with stirring;
method II: dissolving the compound of formula 1 in 80% acetone/water, adding methanol/water (V/V = 4:3), and stirring;
method III: adding the crystal form E of the compound of formula 1 to a solvent IV, and stirring, wherein the solvent IV is selected from the group consisting of one or more of isopropyl ether, methyl *tert*-butyl ether, cyclohexane, isopropyl acetate, and water.

The present disclosure provides a crystal form H of the compound represented by formula 1, wherein an X-ray powder diffraction pattern expressed in terms of 2*θ* angles, which are diffraction angles, of the crystal form has characteristic peaks at 7.961, 13.533, 15.180, 15.879, and 20.619.

In some embodiments, the X-ray powder diffraction pattern expressed in terms of 2*θ* angles, which are diffraction angles, of the crystal form H of the compound represented by formula 1 has characteristic peaks at 7.961, 13.533, 15.180, 15.879, 15.946, 20.619, 20.902, 22.595, and 25.444.

In some embodiments, the X-ray powder diffraction pattern expressed in terms of 2*θ* angles, which are diffraction angles, of the crystal form H of the compound represented by formula 1 has characteristic peaks at 7.961, 13.533, 15.180, 15.879, 15.946, 20.619, 20.902, 22.595, 25.444, 29.305, and 30.671.

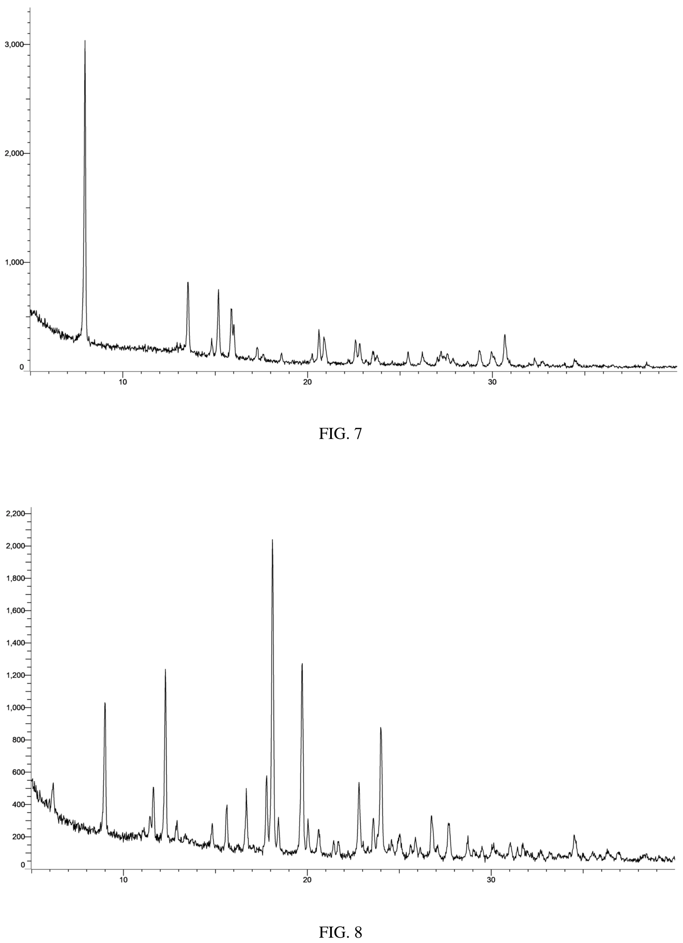

In some embodiments, the X-ray powder diffraction pattern expressed in terms of 2*θ* angles, which are diffraction angles, of the crystal form H of the compound represented by formula 1 is shown in FIG. 7.

The present disclosure further provides a method for preparing the crystal form H of the compound represented by formula 1, the method comprising steps of adding the compound of formula 1 to diethyl ether and slurrying.

In some embodiments, the method for preparing the crystal form H of the compound represented by formula 1 of the present disclosure further comprises steps of adding the crystal form E of the compound of formula 1 to diethyl ether and slurrying.

The present disclosure provides a crystal form I of the compound represented by formula 1, wherein an X-ray powder diffraction pattern expressed in terms of 2*θ* angles, which are diffraction angles, of the crystal form has characteristic peaks at 8.996, 12.286, 18.112, 19.719, and 24.005.

In some embodiments, the X-ray powder diffraction pattern expressed in terms of 2*θ* angles, which are diffraction angles, of the crystal form I of the compound represented by formula 1 has characteristic peaks at 8.996, 12.286, 15.616, 16.690, 18.112, 19.719, 22.812, and 24.005.

In some embodiments, the X-ray powder diffraction pattern expressed in terms of 2*θ* angles, which are diffraction angles, of the crystal form I of the compound represented by formula 1 has characteristic peaks at 8.996, 12.286, 15.616, 16.690, 17.786, 18.112, 19.719, 22.812, 24.005, 26.757, and 27.698.

In some embodiments, the X-ray powder diffraction pattern expressed in terms of 2*θ* angles, which are diffraction angles, of the crystal form I of the compound represented by formula 1 is shown in FIG. 8.

The present disclosure further provides a method for preparing the crystal form I of the compound represented by formula 1, the method comprising steps of adding the compound of formula 1 to n-pentanol and slurrying.

The present disclosure provides a crystal form J of the compound represented by formula 1, wherein an X-ray powder diffraction pattern expressed in terms of 2*θ* angles, which are diffraction angles, of the crystal form has characteristic peaks at 8.928, 12.055, 17.726, 19.533, 22.991, and 23.657.

In some embodiments, the X-ray powder diffraction pattern expressed in terms of 2*θ* angles, which are diffraction angles, of the crystal form J of the compound represented by formula 1 has characteristic peaks at 8.928, 11.725, 12.055, 16.671, 17.726, 19.533, 22.991, 23.657, and 26.826.

In some embodiments, the X-ray powder diffraction pattern expressed in terms of 2*θ* angles, which are diffraction angles, of the crystal form J of the compound represented by formula 1 has characteristic peaks at 6.024, 8.928, 11.725, 12.055, 15.762, 16.671, 17.726, 19.533, 22.991, 23.657, 26.826, and 27.446.

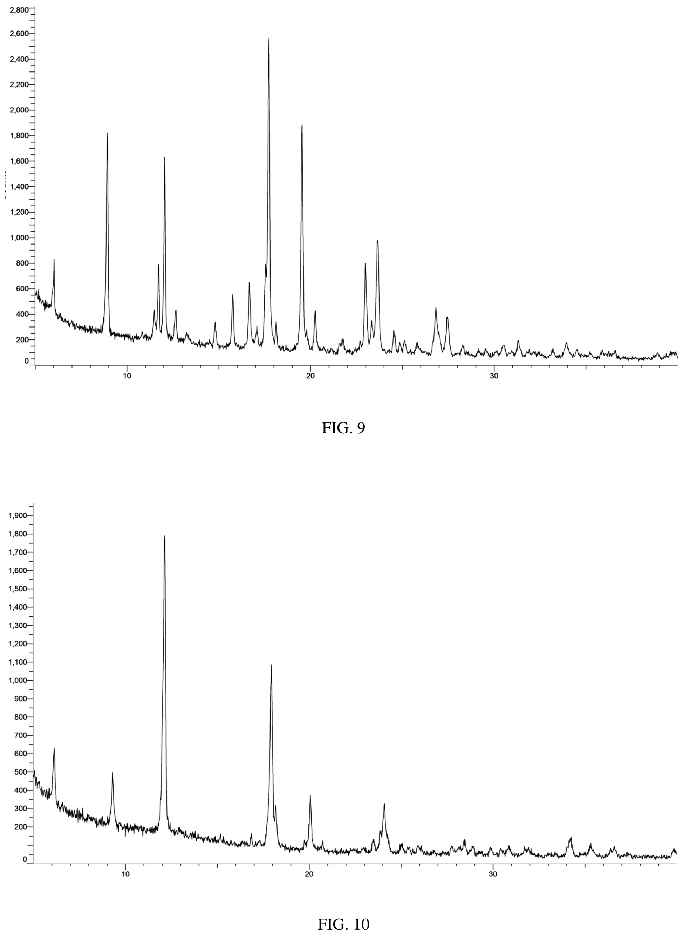

In some embodiments, the X-ray powder diffraction pattern expressed in terms of 2*θ* angles, which are diffraction angles, of the crystal form J of the compound represented by formula 1 is shown in FIG. 9.

The present disclosure further provides a method for preparing the crystal form J of the compound represented by formula 1, the method comprising steps of adding the compound of formula 1 to isopentanol and slurrying.

The present disclosure provides a crystal form K of the compound represented by formula 1, wherein an X-ray powder diffraction pattern expressed in terms of 2*θ* angles, which are diffraction angles, of the crystal form has characteristic peaks at 9.306, 12.132, 17.933, and 20.058.

In some embodiments, the X-ray powder diffraction pattern expressed in terms of 2*θ* angles, which are diffraction angles, of the crystal form K of the compound represented by formula 1 has characteristic peaks at 6.121, 9.306, 12.132, 17.933, 20.058, 24.077, and 34.192.

In some embodiments, the X-ray powder diffraction pattern expressed in terms of 2*θ* angles, which are diffraction angles, of the crystal form K of the compound represented by formula 1 is shown in FIG. 10.

The present disclosure further provides a method for preparing the crystal form K of the compound represented by formula 1, wherein the method is selected from the group consisting of any one of the following methods:
method I: adding the compound of formula 1 to 7% water/ethanol, and stirring;
method II: dissolving the compound of formula 1 in 1,4-dioxane or 10% water/acetone, adding ethanol, and stirring;
method III: dissolving the compound of formula 1 in ethyl acetate/ethanol (v/v = 1:1), heating and cooling between 50 °C and 5 °C, and stirring;
method IV: dissolving the compound of formula 1 in acetonitrile, heating to 65 °C for dissolution, and cooling.

The present disclosure provides a crystal form L of the compound represented by formula 1, wherein an X-ray powder diffraction pattern expressed in terms of 2*θ* angles, which are diffraction angles, of the crystal form has characteristic peaks at 10.645, 16.725, 17.050, 19.513, and 19.780.

In some embodiments, the X-ray powder diffraction pattern expressed in terms of 2*θ* angles, which are diffraction angles, of the crystal form L of the compound represented by formula 1 has characteristic peaks at 6.580, 10.645, 14.011, 14.586, 16.725, 17.050, 19.513, 19.780, and 21.862.

In some embodiments, the X-ray powder diffraction pattern expressed in terms of 2*θ* angles, which are diffraction angles, of the crystal form L of the compound represented by formula 1 has characteristic peaks at 6.580, 10.645, 14.011, 14.586, 16.725, 17.050, 19.513, 19.780, 21.862, 26.362, 26.952, and 27.932.

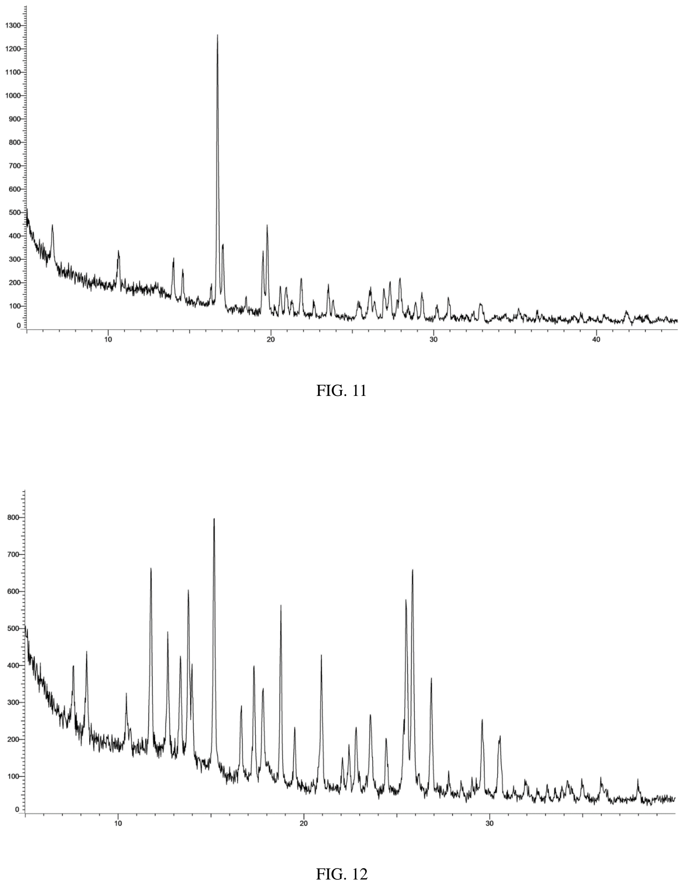

In some embodiments, the X-ray powder diffraction pattern expressed in terms of 2*θ* angles, which are diffraction angles, of the crystal form L of the compound represented by formula 1 is shown in FIG. 11.

The present disclosure further provides a method for preparing the crystal form L of the compound represented by formula 1, the method comprising steps of adding the crystal form F of the compound of formula 1 to water and stirring at 95 °C.

The present disclosure provides a crystal form M of the compound represented by formula 1, wherein an X-ray powder diffraction pattern expressed in terms of 2*θ* angles, which are diffraction angles, of the crystal form has characteristic peaks at 8.313, 11.772, 15.173, 18.762, 20.945, and 25.849.

In some embodiments, the X-ray powder diffraction pattern expressed in terms of 2*θ* angles, which are diffraction angles, of the crystal form M of the compound represented by formula 1 has characteristic peaks at 7.595, 8.313, 11.772, 12.680, 13.791, 15.173, 17.320, 18.762, 20.945, 25.849, and 26.857.

In some embodiments, the X-ray powder diffraction pattern expressed in terms of 2*θ* angles, which are diffraction angles, of the crystal form M of the compound represented by formula 1 has characteristic peaks at 7.595, 8.313, 11.772, 12.680, 13.359, 13.791, 15.173, 16.626, 17.320, 18.762, 19.506, 20.945, 25.849, 26.857, 29.605, and 30.545.

In some embodiments, the X-ray powder diffraction pattern expressed in terms of 2*θ* angles, which are diffraction angles, of the crystal form M of the compound represented by formula 1 is shown in FIG. 12.

The present disclosure further provides a method for preparing the crystal form M of the compound represented by formula 1, the method comprising:
method I: adding the compound of formula 1 to isopropyl acetate, and stirring;
method II: dissolving the compound of formula 1 in isopropyl acetate, adding n-heptane, and stirring.

The present disclosure provides a crystal form N of the compound represented by formula 1, wherein an X-ray powder diffraction pattern expressed in terms of 2*θ* angles, which are diffraction angles, of the crystal form has characteristic peaks at 6.324, 13.092, 13.626, 14.699, 19.483, 22.429, and 27.407.

In some embodiments, the X-ray powder diffraction pattern expressed in terms of 2*θ* angles, which are diffraction angles, of the crystal form N of the compound represented by formula 1 has characteristic peaks at 6.324, 13.092, 13.626, 14.699, 15.566, 19.483, 20.724, 22.429, 23.287, and 27.407.

In some embodiments, the X-ray powder diffraction pattern expressed in terms of 2*θ* angles, which are diffraction angles, of the crystal form N of the compound represented by formula 1 has characteristic peaks at 6.324, 12.664, 13.092, 13.626, 14.699, 15.566, 19.483, 20.724, 22.429, 23.287, 25.042, 26.840, 27.407, and 30.152.

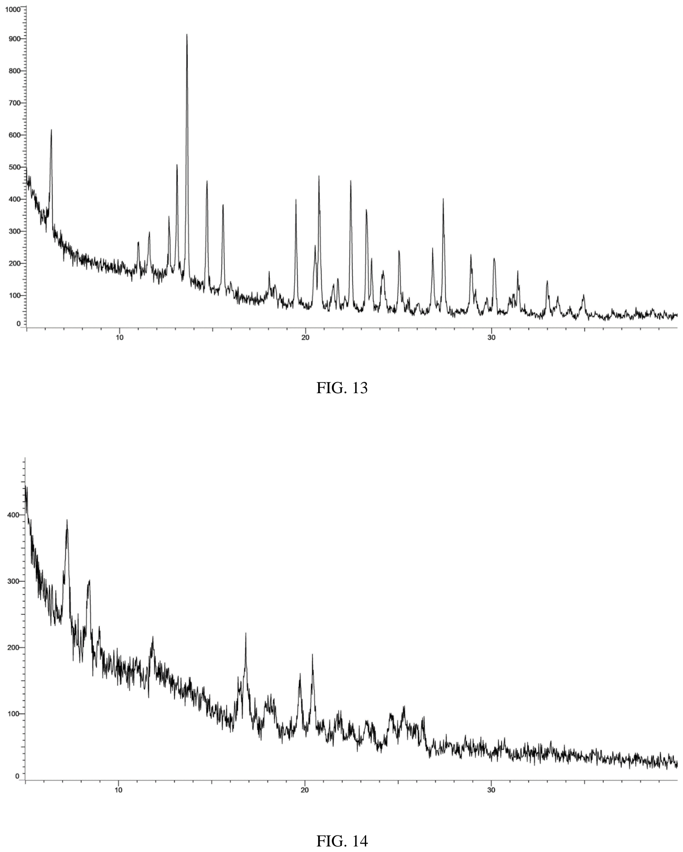

In some embodiments, the X-ray powder diffraction pattern expressed in terms of 2*θ* angles, which are diffraction angles, of the crystal form N of the compound represented by formula 1 is shown in FIG. 13.

The present disclosure further provides a method for preparing the crystal form N of the compound represented by formula 1, the method comprising:
method I: adding the compound of formula 1 to a solvent V, and stirring for crystallization, wherein the solvent V is selected from the group consisting of water and methyl isobutyl ketone;
method II: dissolving the compound of formula 1 in a solvent VI, and adding a seed crystal for crystallization, wherein the solvent VI is selected from the group consisting of one or more of methyl isobutyl ketone and isopropyl acetate;
method III: dissolving the compound of formula 1 in ethyl acetate, adding *n*-heptane, and stirring.

The present disclosure provides a crystal form O of the compound represented by formula 1, wherein an X-ray powder diffraction pattern expressed in terms of 2*θ* angles, which are diffraction angles, of the crystal form has characteristic peaks at 7.224, 8.423, 11.843, 16.829, 19.735, and 20.425.

In some embodiments, the X-ray powder diffraction pattern expressed in terms of 2*θ* angles, which are diffraction angles, of the crystal form O of the compound represented by formula 1 is shown in FIG. 14.

The present disclosure further provides a method for preparing the crystal form O of the compound represented by formula 1, the method comprising: dissolving the compound of formula 1 in 2-butanone, adding water, stirring for 2 h, and centrifuging.

The present disclosure provides a crystal form P of the compound represented by formula 1, wherein an X-ray powder diffraction pattern expressed in terms of 2*θ* angles, which are diffraction angles, of the crystal form has characteristic peaks at 7.848, 16.342, 19.613, 21.305, and 24.786.

In some embodiments, the X-ray powder diffraction pattern expressed in terms of 2*θ* angles, which are diffraction angles, of the crystal form P of the compound represented by formula 1 has characteristic peaks at 7.848, 13.063, 16.342, 19.613, 21.305, 23.584, and 24.786.

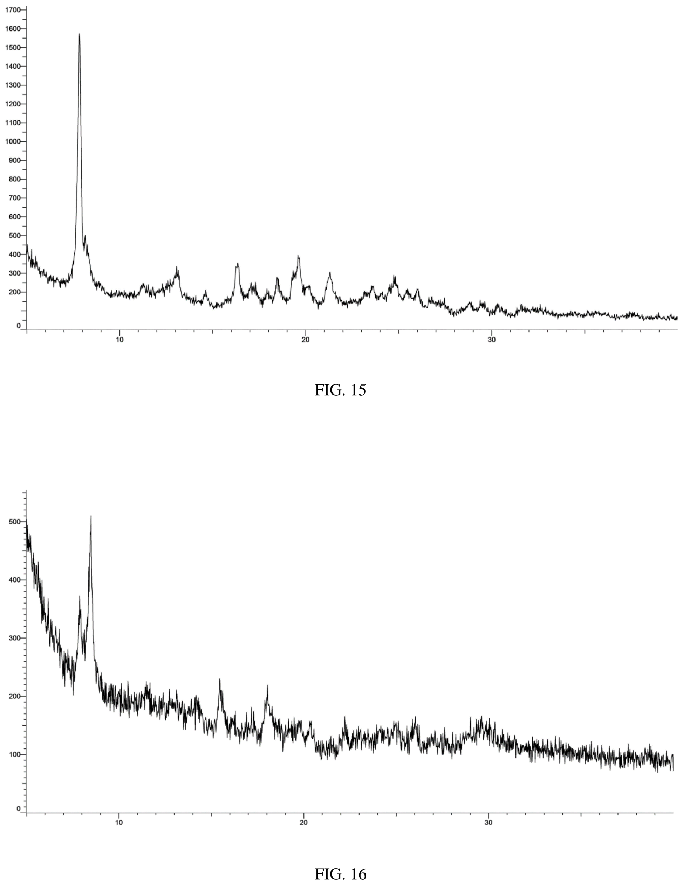

In some embodiments, the X-ray powder diffraction pattern expressed in terms of 2*θ* angles, which are diffraction angles, of the crystal form P of the compound represented by formula 1 is shown in FIG. 15.

The present disclosure further provides a method for preparing the crystal form P of the compound represented by formula 1, the method comprising: steps of dissolving the compound of formula 1 in 2-butanone, adding water, stirring for 24 h, and centrifuging.

The present disclosure provides a crystal form Q of the compound represented by formula 1, wherein an X-ray powder diffraction pattern expressed in terms of 2*θ* angles, which are diffraction angles, of the crystal form has characteristic peaks at 7.921, 8.455, 15.468, 18.035, 22.224, and 26.015.

In some embodiments, the X-ray powder diffraction pattern expressed in terms of 2*θ* angles, which are diffraction angles, of the crystal form Q of the compound represented by formula 1 is shown in FIG. 16.

The present disclosure further provides a method for preparing the crystal form Q of the compound represented by formula 1, the method comprising: steps of dissolving the compound of formula 1 in 2-butanone, adding water, stirring for 2 h, centrifuging, and drying.

The present disclosure provides a crystal form R of the compound represented by formula 1, wherein an X-ray powder diffraction pattern expressed in terms of 2*θ* angles, which are diffraction angles, of the crystal form has characteristic peaks at 9.598, 11.283, 13.705, 15.045, 19.714, and 24.020.

In some embodiments, the X-ray powder diffraction pattern expressed in terms of 2*θ* angles, which are diffraction angles, of the crystal form R of the compound represented by formula 1 has characteristic peaks at 8.248, 9.598, 11.283, 12.828, 13.705, 15.045, 15.539, 19.714, 22.430, 24.020, and 26.248.

In some embodiments, the X-ray powder diffraction pattern expressed in terms of 2*θ* angles, which are diffraction angles, of the crystal form R of the compound represented by formula 1 has characteristic peaks at 8.248, 9.598, 9.884, 11.283, 11.748, 12.828, 13.705, 15.045, 15.539, 16.670, 17.686, 19.714, 20.791, 22.430, 24.020, 25.017, and 26.248.

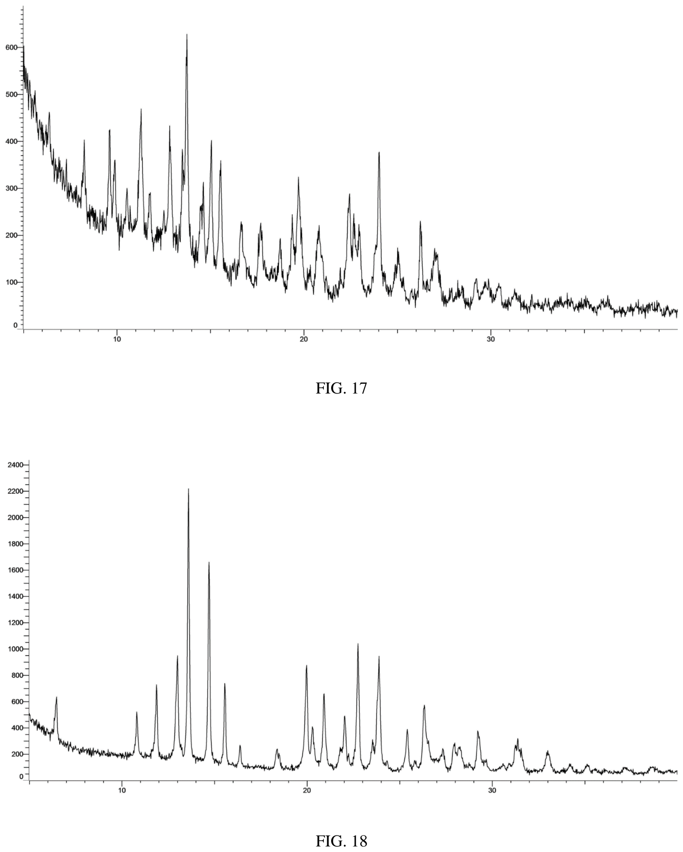

In some embodiments, the X-ray powder diffraction pattern expressed in terms of 2*θ* angles, which are diffraction angles, of the crystal form R of the compound represented by formula 1 is shown in FIG. 17.

The present disclosure further provides a method for preparing the crystal form R of the compound represented by formula 1, the method comprising:
step I: dissolving the compound of formula 1 in 2-butanone, adding water, heating to 50 °C, and stirring for crystallization; and
step II: adding the crystal obtained in step I to water, and stirring at 60 °C for crystallization.

The present disclosure provides a crystal form S of the compound represented by formula 1, wherein an X-ray powder diffraction pattern expressed in terms of 2*θ* angles, which are diffraction angles, of the crystal form has characteristic peaks at 10.810, 13.597, 14.706, 19.971, and 22.751.

In some embodiments, the X-ray powder diffraction pattern expressed in terms of 2*θ* angles, which are diffraction angles, of the crystal form S of the compound represented by formula 1 has characteristic peaks at 6.465, 10.810, 11.872, 13.597, 14.706, 15.563, 19.971, 22.751, 23.881, and 26.322.

In some embodiments, the X-ray powder diffraction pattern expressed in terms of 2*θ* angles, which are diffraction angles, of the crystal form S of the compound represented by formula 1 has characteristic peaks at 6.465, 10.810, 11.872, 12.996, 13.597, 14.706, 15.563, 16.385, 19.971, 20.914, 22.751, 23.881, 25.414, 26.322, 29.235, and 32.963.

In some embodiments, the X-ray powder diffraction pattern expressed in terms of 2*θ* angles, which are diffraction angles, of the crystal form S of the compound represented by formula 1 is shown in FIG. 18.

The present disclosure further provides a method for preparing the crystal form S of the compound represented by formula 1, the method comprising:
step I: adding the compound of formula 1 to methyl isobutyl ketone, and stirring for crystallization; and
step II: drying the crystal obtained in step I *in vacuo* at 100 °C.

The present disclosure provides a crystal form T of the compound represented by formula 1, wherein an X-ray powder diffraction pattern expressed in terms of 2*θ* angles, which are diffraction angles, of the crystal form has characteristic peaks at 6.431, 19.884, 21.207, 21.993, 24.138, 24.924, and 25.111.

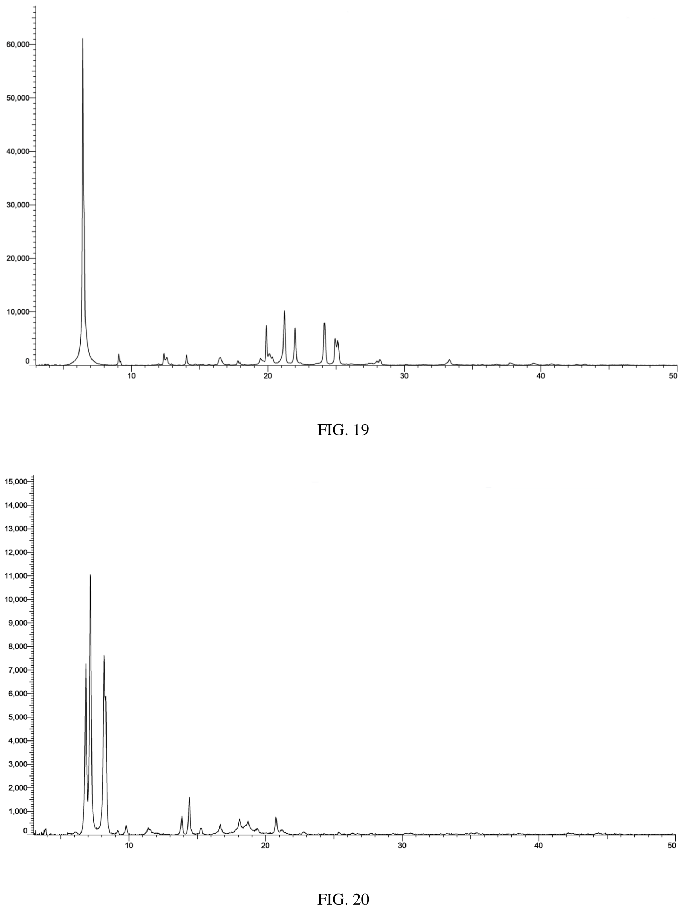

In some embodiments, the X-ray powder diffraction pattern expressed in terms of 2*θ* angles, which are diffraction angles, of the crystal form T of the compound represented by formula 1 is shown in FIG. 19.

The present disclosure further provides a method for preparing the crystal form T of the compound represented by formula 1, the method comprising steps of dissolving the compound of formula 1 in isopropanol and evaporating the solvent.

The present disclosure provides a crystal form U of the compound represented by formula 1, wherein an X-ray powder diffraction pattern expressed in terms of 2*θ* angles, which are diffraction angles, of the crystal form has characteristic peaks at 6.843, 7.185, 8.193, 13.870, 14.416, and 20.769.

In some embodiments, the X-ray powder diffraction pattern expressed in terms of 2*θ* angles, which are diffraction angles, of the crystal form U of the compound represented by formula 1 has characteristic peaks at 6.843, 7.185, 8.193, 13.870, 14.416, 16.683, 18.102, 18.721, and 20.769.

In some embodiments, the X-ray powder diffraction pattern expressed in terms of 2*θ* angles, which are diffraction angles, of the crystal form U of the compound represented by formula 1 is shown in FIG. 20.

The present disclosure further provides a method for preparing the crystal form U of the compound represented by formula 1, the method comprising steps of adding the compound of formula 1 to 90% methanol/water and slurrying.

The present disclosure provides a crystal form B of the compound represented by formula 1, wherein an X-ray powder diffraction pattern expressed in terms of 2*θ* angles, which are diffraction angles, of the crystal form has characteristic peaks at 8.587, 10.089, 11.875, 16.987, 21.717, and 23.436.

In some embodiments, the X-ray powder diffraction pattern expressed in terms of 2*θ* angles, which are diffraction angles, of the crystal form B of the compound represented by formula 1 has characteristic peaks at 7.545, 8.587, 10.089, 11.117, 11.875, 16.601, 16.987, 17.056, 18.838, 19.285, 21.287, 21.717, and 23.436.

In some embodiments, the X-ray powder diffraction pattern expressed in terms of 2*θ* angles, which are diffraction angles, of the crystal form B of the compound represented by formula 1 has characteristic peaks at 7.545, 8.587, 10.089, 11.117, 11.875, 15.809, 16.601, 16.987, 17.056, 18.397, 18.515, 18.838, 19.285, 21.287, 21.717, 22.527, 22.881, 23.436, 26.541, 29.276, and 30.437.

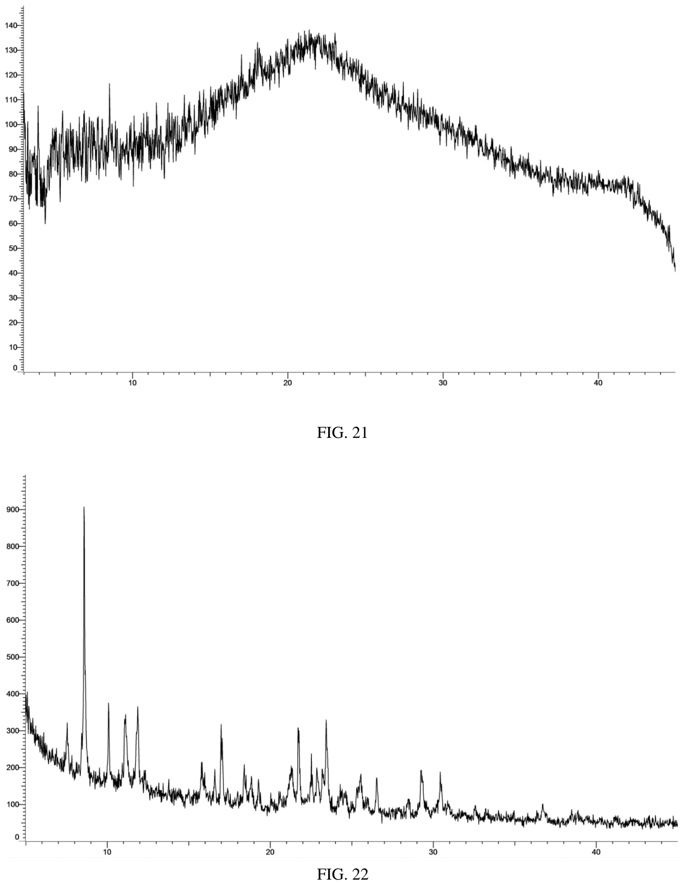

In some embodiments, the X-ray powder diffraction pattern expressed in terms of 2*θ* angles, which are diffraction angles, of the crystal form B of the compound represented by formula 1 is shown in FIG. 22.

The present disclosure further provides a method for preparing the crystal form B of the compound represented by formula 1, the method comprising steps of adding the crystal form E of the compound of formula 1 to ethyl acetate and stirring.

The present disclosure provides a crystal form α of the compound represented by formula 1, wherein an X-ray powder diffraction pattern expressed in terms of 2*θ* angles, which are diffraction angles, of the crystal form has characteristic peaks at 6.622, 9.944, 11.103, 17.932, 22.143, and 25.120.

In some embodiments, the X-ray powder diffraction pattern expressed in terms of 2*θ* angles, which are diffraction angles, of the crystal form α of the compound represented by formula 1 has characteristic peaks at 6.622, 9.944, 11.103, 15.927, 17.932, 20.112, 22.143, 23.602, 25.120, and 29.026.

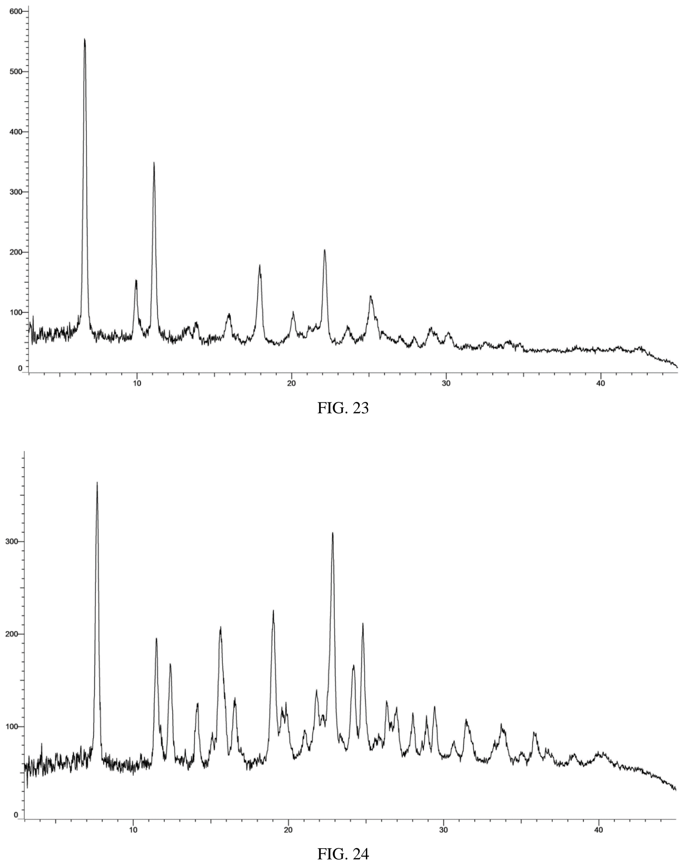

In some embodiments, the X-ray powder diffraction pattern expressed in terms of 2*θ* angles, which are diffraction angles, of the crystal form α of the compound represented by formula 1 is shown in FIG. 23.

The present disclosure further provides a method for preparing the crystal form α of the compound represented by formula 1, the method comprising adding the crystal form N of the compound of formula 1 to a solvent c and stirring, wherein the solvent c is selected from the group consisting of one or more of methanol, 50% methanol/water, 7% water/ethanol, and 50% acetonitrile/methanol.

The present disclosure provides a crystal form β of the compound represented by formula 1, wherein an X-ray powder diffraction pattern expressed in terms of 2*θ* angles, which are diffraction angles, of the crystal form has characteristic peaks at 7.683, 12.400, 15.616, 19.040, 22.856, and 24.802.

In some embodiments, the X-ray powder diffraction pattern expressed in terms of 2*θ* angles, which are diffraction angles, of the crystal form β of the compound represented by formula 1 has characteristic peaks at 7.683, 11.501, 12.400, 14.114, 15.616, 16.527, 19.040, 22.856, 24.200, 24.802, 26.963, and 29.420.

In some embodiments, the X-ray powder diffraction pattern expressed in terms of 2*θ* angles, which are diffraction angles, of the crystal form β of the compound represented by formula 1 has characteristic peaks at 7.683, 11.501, 12.400, 14.114, 15.616, 16.527, 19.040, 19.592, 19.861, 21.815, 22.856, 24.200, 24.802, 26.963, 28.023, 29.420, 31.500, and 33.726.

In some embodiments, the X-ray powder diffraction pattern expressed in terms of 2*θ* angles, which are diffraction angles, of the crystal form β of the compound represented by formula 1 is shown in FIG. 24.

The present disclosure further provides a method for preparing the crystal form β of the compound represented by formula 1, the method comprising steps of adding the crystal form B of the compound of formula 1 to propylene glycol methyl ether and stirring.

The present disclosure further provides a pharmaceutical composition comprising any one or more of the aforementioned crystal forms A to U, crystal form α, or crystal form β, and optionally a pharmaceutically acceptable auxiliary material selected from the group consisting of pharmaceutically acceptable excipients.

The present disclosure further provides a pharmaceutical composition prepared from any one or more of the aforementioned crystal forms A to U, crystal form α, or crystal form β, and optionally a pharmaceutically acceptable excipient.

The present disclosure further provides a method for preparing a pharmaceutical composition, the method comprising a step of mixing any one or more of the aforementioned crystal forms A to U, crystal form α, or crystal form β with a pharmaceutically acceptable excipient.

The present disclosure further provides use of any one or more of the aforementioned crystal forms A to U, crystal form α, or crystal form β, or the aforementioned composition in the manufacture of a medicament for preventing and/or treating a tumor.

Provided is the use described in the present disclosure, wherein the tumor is selected from the group consisting of breast cancer, ovarian cancer, pancreatic cancer, prostate cancer, gastric cancer, colorectal cancer, lung cancer, renal cancer, hepatic cancer, cervical cancer, endometrial cancer, myeloma, leukemia, lymphoma, acoustic neuroma, basal cell carcinoma, cholangiocarcinoma, bladder cancer, brain cancer, bronchogenic carcinoma, sarcoma, chordoma, choriocarcinoma, craniopharyngioma, cystadenocarcinoma, embryonal carcinoma, hemangioendothelioma, ependymoma, epithelial cancer, esophageal cancer, essential thrombocytosis, Ewing sarcoma, testicular cancer, glioma, heavy chain disease, hemangioblastoma, medullary carcinoma, medulloblastoma, melanoma, meningioma, mesothelioma, neuroblastoma, NUT midline carcinoma, neuroglioma, bone cancer, na-sopharyngeal carcinoma, oral cancer, thyroid cancer, pinealoma, polycythemia vera, ret-inoblastoma, sebaceous carcinoma, seminoma, skin cancer, squamous cell carcinoma, synovioma, sweat gland carcinoma, Waldenström macroglobulinemia, and Wilms tumor; preferably, the tumor is selected from the group consisting of breast cancer, ovarian cancer, pancreatic cancer, prostate cancer, gastric cancer, colorectal cancer, and lung cancer. As used herein, the "2*θ* or 2*θ* angle" refers to a diffraction angle; *θ* refers to the Bragg angle in ° or degrees; the 2*θ* of each characteristic peak has a margin of error of ±0.20 (including the case that a number having more than 1 decimal place is rounded), specifically -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, numerical values such as the content of related substances are data obtained by measurement and calculation, and there is inevitably a certain degree of error. In general, ±10% is a reasonable margin of error. There is a certain degree of error change depending on the context where it is used, and the error change is no more than ±10% and may be ±9%, ±8%, ±7%, ±6%, ±5%, ±4%, ±3%, ±2%, or ±1%, preferably ±5%.

The starting material used in the methods for preparing the crystal forms of the present disclosure may be any form of the compound, and the specific forms include, but are not limited to: an amorphous form, any crystal form, a hydrate, a solvate, and the like.

In the present disclosure, the drying temperature is generally 25 °C-100 °C, preferably 40 °C-70 °C, and the drying may be performed under atmospheric pressure or reduced pressure.

Methods for the crystallization described in the present disclosure include room-temperature crystallization, cooling crystallization, crystallization by volatilizing the solvent, crystallization induced by adding a seed crystal, and the like, wherein the cooling temperature is selected from the group consisting of 65 °C or lower, preferably from the group consisting of -10 °C to 60 °C, and the crystallization process may involve stirring.

As used herein, the "differential scanning calorimetry" or "DSC" refers to the measurement of the temperature difference and heat flow difference between a sample and a reference substance during a ramping or thermostatic process to characterize all the physical changes and chemical changes related to the thermal effect, and to acquire phase change information about the sample.

According to the description of hygroscopicity characteristics and the definitions of hygroscopic weight gains in "General Rule 9103, Guidelines for Hygroscopicity Trials for Pharmaceuticals" in the Chinese Pharmacopoeia, Volume IV, 2015 Edition,
deliquescent: sufficient water is absorbed to form a liquid;
extremely hygroscopic: the hygroscopic weight gain is not less than 15%;
hygroscopic: the hygroscopic weight gain is less than 15% but not less than 2%;
slightly hygroscopic: the hygroscopic weight gain is less than 2% but not less than 0.2%; nonhygroscopic or practically nonhygroscopic: the hygroscopic weight gain is less than 0.2%.

As used herein, the "excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 shows an XRPD pattern of the crystal form A of compound 1.
FIG. 2 shows an XRPD pattern of the crystal form C of compound 1.
FIG. 3 shows an XRPD pattern of the crystal form D of compound 1.
FIG. 4 shows an XRPD pattern of the crystal form E of compound 1.
FIG. 5 shows an XRPD pattern of the crystal form F of compound 1.
FIG. 6 shows an XRPD pattern of the crystal form G of compound 1.
FIG. 7 shows an XRPD pattern of the crystal form H of compound 1.
FIG. 8 shows an XRPD pattern of the crystal form I of compound 1.
FIG. 9 shows an XRPD pattern of the crystal form J of compound 1.
FIG. 10 shows an XRPD pattern of the crystal form K of compound 1.
FIG. 11 shows an XRPD pattern of the crystal form L of compound 1.
FIG. 12 shows an XRPD pattern of the crystal form M of compound 1.
FIG. 13 shows an XRPD pattern of the crystal form N of compound 1.
FIG. 14 shows an XRPD pattern of the crystal form O of compound 1.
FIG. 15 shows an XRPD pattern of the crystal form P of compound 1.
FIG. 16 shows an XRPD pattern of the crystal form Q of compound 1.
FIG. 17 shows an XRPD pattern of the crystal form R of compound 1.
FIG. 18 shows an XRPD pattern of the crystal form S of compound 1.
FIG. 19 shows an XRPD pattern of the crystal form T of compound 1.
FIG. 20 shows an XRPD pattern of the crystal form U of compound 1.
FIG. 21 shows an XRPD pattern of the amorphous form of compound 1.
FIG. 22 shows an XRPD pattern of the crystal form B of compound 1.
FIG. 23 shows an XRPD pattern of the crystal form α of compound 1.
FIG. 24 shows an XRPD pattern of the crystal form β of compound 1.

### DETAILED DESCRIPTION

The present disclosure is explained below in greater detail with reference to examples or experimental examples. The examples or experimental examples in the present disclosure are only illustrative of the technical solutions in the present disclosure and do not limit the spirit and scope of the present disclosure.

Test conditions for the instruments used in the experiments:
The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE NEO 500M nuclear magnetic resonance system, with dimethyl sulfoxide-D6 (DMSO-*d*₆), chloroform-D (CDCl₃), and methanol-D4 (CD₃OD) as solvents and tetramethylsilane (TMS) as an internal standard.
The MS analyses were performed using an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters; MS model: Waters ACQuity Qda Detector/Waters SQ Detector), and THERMO Ultimate 3000-Q Exactive (manufacturer: THERMO; MS model: THERMO Q Exactive). The high performance liquid chromatography (HPLC) analyses were performed using Agilent HPLC 1200DAD, Agilent HPLC 1200VWD, and Waters HPLC e2695-2489 high performance liquid chromatographs.

The preparative high performance liquid chromatography was performed using Waters 2767, Waters 2767-SQ Detector 2, Shimadzu LC-20AP, and Gilson-281 preparative chromatographs.

The preparative chiral chromatography was performed using a Shimadzu LC-20AP preparative chromatograph.

The CombiFlash preparative flash chromatograph used was Combiflash Rf200 (TELE-DYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm. The silica gel column chromatography generally used Yantai Huanghai 200-300 mesh silica gel as the carrier.

The mean kinase inhibition rates and IC50 values were measured using a NovoStar microplate reader (BMG, Germany).

The known starting materials in the present disclosure may be synthesized by using or according to methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed in an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenation apparatus and a Qinglan QL-500 hydrogen generator, or an HC2-SS hydrogenation apparatus.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of reaction progress in the examples was performed using thin-layer chromatography (TLC). The developing solvents used in the reactions, the eluent systems used in the column chromatography purification of compounds, and the developing solvent systems used in the thin-layer chromatography include: A: petroleum ether/ethyl acetate system, and B: dichloromethane/methanol system. The volume ratio of the solvents was adjusted depending on the polarity of a compound, or by adding a small amount of a basic or acidic reagent such as triethylamine and acetic acid.

XRPD refers to X-ray powder diffraction: The analysis was performed using a BRUKER D8 X-ray diffractometer; specific acquisition information: a Cu anode (40 kV, 40 mA), ray: monochromatic Cu-Ka ray (λ = 1.5418 Å). Scan mode: *θ*/2*θ*, scan range (2*θ* range): 3° to 45°.

DSC refers to differential scanning calorimetry: The analysis was performed using a METTLER TOLEDO DSC 3+ differential scanning calorimeter, with a temperature ramp rate of 10 °C/min, specific temperature ranges shown in corresponding thermograms (mostly 25-350 °C), and a nitrogen purge speed of 50 mL/min.

TGA refers to thermogravimetric analysis: The analysis was performed using a METTLER TOLEDO TGA 2 thermogravimetric analyzer, with a temperature ramp rate of 10 °C/min, specific temperature ranges shown in corresponding thermograms (mostly 25-350 °C), and a nitrogen purge speed of 50 mL/min.

DVS refers to dynamic vapor sorption: The analysis was performed using SMS DVS Advantage; at 25 °C, the humidity was changed as follows: 50%-95%-0%-95%-50%, in steps of 10% (5% for the last step) (specific humidity ranges are shown in corresponding patterns, and those listed here were used in most cases); the criterion was dm/dt being not greater than 0.002%.

### Example 1: Preparation of Compound of Formula 1

### (S)-4-((S)-10-Acryloyl-4-chloro-2-fluoro-14-oxo-8,8a,9,10,11,12-hexahydro-7H,14H-pyrazino[1',2':5,6][1,5]diazocino[3,2,1-hi]indazol-3-yl)-2-amino-7-fluorobenzo[b]thiophene-3-carbonitrile 1 (with reference to the preparation method of Example 7-P2 in Application No. PCT/CN2022/126650)

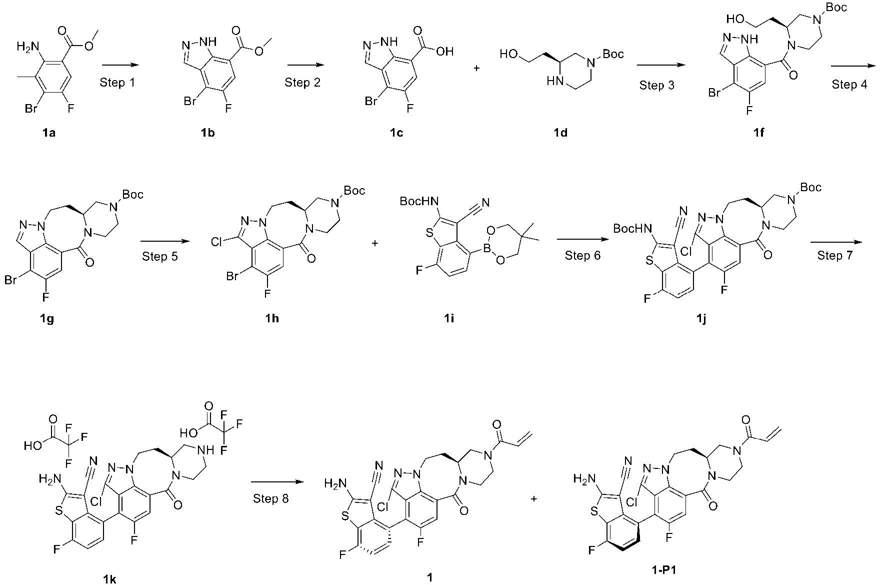

### Step 1

### Methyl 4-bromo-5-fluoro-1H-indazole-7-carboxylate 1b

Methyl 2-amino-4-bromo-5-fluoro-3-methylbenzoate **1a** (2.7 g, 10.3 mmol, prepared using the method disclosed in step (iv) on page 103 of the specification of the patent application "WO2016020836A1") was dissolved in chloroform (50 mL), and acetic anhydride (3.16 g, 30.9 mmol) was added. After the mixture was stirred for 90 min with the temperature maintained below 40 °C, potassium acetate (302 mg, 3.08 mmol) and *tert*-butyl nitrite (2.12 g, 20.6 mmol) were added. The mixture was refluxed for 14 h. The reaction mixture was cooled to room temperature, diluted with dichloromethane (50 mL), and washed with water (30 mL), a saturated sodium carbonate solution (10 mL), and a saturated sodium chloride solution (10 mL) in sequence. The organic phase was dried over anhydrous sodium sulfate and filtered. The filtrate was concentrated under reduced pressure to give a crude product of the title compound **1b** (2.8 g). The product was directly used in the next step without purification.

MS m/z (ESI): 272.9 [M+1].

### Step 2

### 4-Bromo-5-fluoro-1H-indazole-7-carboxylic acid 1c

The crude product of compound **1b** (2.8 g, 10.25 mmol) was dissolved in a mixed solvent of tetrahydrofuran (20 mL), methanol (10 mL), and water (10 mL), and lithium hydroxide (2.15 g, 51.26 mmol) was added. The mixture was stirred at 40 °C for 1 h. The reaction mixture was concentrated under reduced pressure to remove most of the solvent. Water was added, and extraction was performed with ethyl acetate (150 mL × 6). The organic phases were combined, washed with dilute hydrochloric acid and a saturated sodium chloride solution in sequence, and dried over anhydrous sodium sulfate, and then the drying agent was removed by filtration. The filtrate was concentrated under reduced pressure to give a crude product of compound **1c** (1.4 g). The product was directly used in the next step without purification.

MS m/z (ESI): 259.0 [M+1].

### Step 3

### tert-Butyl (S)-4-(4-bromo-5-fluoro-1H-indazole-7-carbonyl)-3-(2-hydroxyethyl)piperazine-1-carboxylate 1f

The crude product of compound **1c** (600 mg, 2.32 mmol) and *tert*-butyl (*S*)-3-(2-hydroxyethyl)piperazine-1-carboxylate **1d** (586 mg, 2.54 mmol, prepared using the method disclosed in preparation 65 on page 80 of the specification of the patent application "WO2021118877A1") were dissolved in 30 mL of *N,N*-dimethylformamide, and *N,N*-diisopropylethylamine (898 mg, 6.95 mmol) and *O*-(7-azabenzotriazol-1-yl)-*N,N,N',N*'-tetramethyluronium hexafluorophosphate (1.05 g, 2.78 mmol) were added in an ice bath. The mixture was stirred for 1 h. The reaction mixture was concentrated under reduced pressure, then diluted with ethyl acetate, and washed with water, a saturated sodium carbonate solution, and a saturated sodium chloride solution in sequence. The organic phase was dried over anhydrous sodium sulfate and filtered. The filtrate was concentrated under reduced pressure, and the residue was purified by silica gel column chromatography with eluent system A to give the title compound **1f** (600 mg, yield: 54.9%). MS m/z (ESI): 415.2 [M-55].

### Step 4

### tert-Butyl (S)-3-bromo-2-fluoro-14-oxo-7,8,8a,9,11,12-hexahydro-10H,14H-pyrazino[1',2':5,6][1,5]diazocino[3,2,1-hi]indazole-10-carboxylate 1g

Compound **1f** (600 mg, 1.27 mmol) was dissolved in 36 mL of tetrahydrofuran in a nitrogen atmosphere, and triphenylphosphine (667 mg, 2.54 mmol) and diethyl azodicarboxylate (514 mg, 2.54 mmol) were added in sequence in an ice bath. The mixture was stirred for 30 min with the temperature maintained. The reaction mixture was quenched with a saturated ammonium chloride solution and extracted with ethyl acetate (15 mL × 2). The organic phases were combined, washed with a saturated sodium chloride solution, dried over anhydrous sodium sulfate, and filtered. The filtrate was concentrated under reduced pressure, and the residue was purified by silica gel column chromatography with eluent system A to give the title compound **1g** (577 mg, yield: 99.9%).

MS m/z (ESI): 397.2 [M-55].

### Step 5

### tert-Butyl (S)-3-bromo-4-chloro-2-fluoro-14-oxo-7,8,8a,9,11,12-hexahydro-10H,14H-pyrazino[1',2':5,6][1,5]diazocino[3,2,1-hi]indazole-10-carboxylate 1h

Compound **1g** (630 mg, 1.38 mmol) was dissolved in acetonitrile (6 mL), and *N-*chlorosuccinimide (278 mg, 2.08 mmol) was added. The mixture was heated to 60 °C and left to react for 0.5 h, and a solid was precipitated. An additional amount of *N*-chlorosuccinimide (100 mg, 0.75 mmol) was added, and the mixture was left to react for another 20 min. The reaction mixture was cooled to room temperature. A saturated sodium bicarbonate solution was added, and extraction was performed with ethyl acetate (10 mL × 3). The organic phases were combined and dried over anhydrous sodium sulfate, and then the drying agent was removed by filtration. The filtrate was concentrated under reduced pressure, and the residue was purified by silica gel column chromatography with eluent system A to give the title compound **1h** (380 mg, yield: 56%).

MS m/z (ESI): 487.1 [M+1].

### Step 6

### tert-Butyl (8aS)-3-(2-((tert-butoxycarbonyl)amino)-3-cyano-7-fluorobenzo[b]thiophen-4-yl)-4-chloro-2-fluoro-14-oxo-7,8,8a,9,11,12-hexahydro-10H,14H-pyrazino[1',2':5,6][1,5]diazocino[3,2,1-hi]indazole-10-carboxylate 1j

Compound **1h** (150 mg, 0.31 mmol) and compound **1i** (186 mg, 0.46 mmol, prepared using the method disclosed in preparation 15 on page 50 of the specification of the patent application "WO2021118877A1") were dissolved in 5 mL of toluene, and di-chloro[bis(diphenylphosphinophenyl)ether]palladium(II) (43 mg, 0.06 mmol) and cesium carbonate (300 mg, 0.92 mmol) were added. The system was purged with nitrogen gas, and the mixture was stirred at 105 °C for 6 h. The reaction mixture was cooled to room temperature and then filtered. The filtrate was concentrated under reduced pressure, and the residue was purified by silica gel column chromatography with eluent system A to give the title compound **1j** (40 mg, yield: 18.6%).

MS m/z (ESI): 699.2 [M+1].

### Step 7

### 2-Amino-4-((S)-4-chloro-2-fluoro-14-oxo-8,8a,9,10,11,12-hexahydro-7H,14H-pyrazino[1',2':5,6] [1,5]diazocino[3,2,1-hi]indazol-3-yl)-7-fluorobenzo[b]thiophene-3-carbonitrile bis(2,2,2-trifluoroacetate) 1k

Compound **1j** (40 mg, 57.21 µmol) was dissolved in 1 mL of dichloromethane, and 1 mL of trifluoroacetic acid was added at 0 °C. The mixture was stirred for 2 h. The reaction mixture was concentrated under reduced pressure to give a crude product of the title compound **1k** (41 mg). The product was directly used in the next step without purification.

MS m/z (ESI): 499.2 [M+1].

### Step 8

### (S)-4-((S)-10-Acryloyl-4-chloro-2-fluoro-14-oxo-8,8a,9,10,11,12-hexahydro-7H,14H-pyrazino[1',2':5,6][1,5]diazocino[3,2,1-hi]indazol-3-yl)-2-amino-7-fluorobenzo[b]thiophene-3-carbonitrile 1

The crude product of compound **1k** (41 mg, 56.4 µmol) was suspended in 2 mL of ethyl acetate, 1 mL of tetrahydrofuran, and 2 mL of water, and anhydrous potassium carbonate (24 mg, 173.6 µmol) was added. Acryloyl chloride (5.4 mg, 59.6 µmol) was added in an ice bath. After 5 min of reaction, extraction was performed with ethyl acetate (5 mL × 2). The organic phases were combined and concentrated under reduced pressure to give a crude product of compound **1,** i.e., 4-((*S*)-10-acryloyl-4-chloro-2-fluoro-14-oxo-8,8*a*,9,10,11,12-hexahydro-7*H*,14*H*-pyrazino[1',2':5][1,5]diazocino[3,2,1-*hi*]indazol-3-yl)-2-amino-7-fluorobenzo[*b*]thiophene-3-carbonitrile (39 mg). The crude product was purified by preparative high performance liquid chromatography (Waters-2545; column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compound **1** (5 mg, yield: 15.1%). According to X-ray powder diffraction analysis, the product was defined as an amorphous form. Its XRPD pattern is shown in FIG. 21.
Single-configuration compound (shorter retention time) **1** (5 mg, yield: 15.1%)
MS m/z (ESI): 553.2 [M+1].
HPLC analysis: retention time: 2.21 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).
¹H NMR (500 MHz, CD₃OD): δ 7.67 (d, 1H), 7.20 (t, 1H), 7.02 (dd, 1H), 6.87 (dd, 1H), 6.72-6.28 (m, 1H), 5.81 (t, 1H), 4.74-4.67 (m, 1H), 4.66-4.56 (m, 1H), 4.41 (d, 1H), 4.30 (d, -1H), 4.12-4.00 (m, 1H), 3.96 (d, 1H), 3.49 (d, 1H), 3.09 (dd, 2H), 2.26-2.16 (m, 1H), 1.94 (s, 1H).
Single-configuration compound (longer retention time) **1-P1** (2 mg, yield: 6%)
MS m/z (ESI): 553.2 [M+1].
HPLC analysis: retention time: 2.29 min; purity: 95% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).
¹H NMR (500 MHz, CD₃OD): δ 7.65 (d, 1H), 7.20 (t, 1H), 7.00 (dd, 1H), 6.85 (dd, 1H), 6.70-6.26 (m, 1H), 5.81 (t, 1H), 4.73-4.65 (m, 1H), 4.66-4.56 (m, 1H), 4.39 (d, 1H), 4.30 (d, 1H), 4.10-4.00 (m, 1H), 3.92 (d, 1H), 3.49 (d, 1H), 3.12 (dd, 2H), 2.24-2.16 (m, 1H), 1.92 (s, 1H).

### Test Example 1: H358 Cell Proliferation Assay

The inhibitory activity of the compound of the present disclosure against H358 cell proliferation was measured. Below is a brief description of the experimental method:
H358 cells (ATCC, CRL-5807) were cultured in RPMI1640 medium (Hyclone, SH30809.01) containing 10% fetal bovine serum (Corning, 35-076-CV) (i.e., complete medium). On the first day of the experiment, H358 cells were seeded into a 96-well plate at a density of 1200 cells/well using complete medium, with 100 µL of cell suspension per well, and the plate was incubated overnight in a 5% CO₂ cell incubator at 37 °C. On the second day, the test compound, prepared in complete medium and serially diluted, was added at 10 µL/well. The final concentrations of the compound were 9 concentration points obtained by a 5-fold serial dilution from 10 µM. A blank control containing 0.5% DMSO was set up. The plate was incubated for 120 h in a 5% CO₂ cell incubator at 37 °C. On the seventh day, the 96-well cell plate was removed from the incubator, and Cell Titer-Glo^{®} Luminescent Cell Viability Assay (Promega, G7573) was added at 50 µL/well. After the plate was left to stand at room temperature for 10 min, the luminescence signal values were measured using a multifunctional microplate reader (PerkinElmer, EnVision^{®} 2105), and the IC₅₀ value for the inhibitory activity of the compound was calculated using Graphpad Prism software.

**Table 1. The inhibitory activity of the compound of the present disclosure against H358 cell proliferation**

| Compound No. | IC₅₀ (nM) |
|---|---|
| 1 | 0.74 |

| | |
|---|---|
| Conclusion: The compound of the present disclosure has an inhibitory effect on H358 cell proliferation. | |

### Test Example 2: MIA PaCa-2 Cell Proliferation Assay

The inhibitory activity of the compound of the present disclosure against MIA PaCa-2 cell proliferation was measured. Below is a brief description of the experimental method: MIA PaCa-2 cells (ATCC, CRL-1420) were cultured in DMEM/HIGH GLUCOSE medium (GE, SH30243.01) containing 10% fetal bovine serum (Corning, 35-076-CV) and 2.5% horse serum (Beyotime Biological Tech., C0262) (i.e., complete medium). On the first day of the experiment, MIA PaCa-2 cells were seeded into a 96-well plate at a density of 500 cells/well using complete medium, with 90 µL of cell suspension per well, and the plate was incubated overnight in a 5% CO₂ cell incubator at 37 °C. On the second day, the test compound, prepared in complete medium and serially diluted, was added at 10 µL/well. The final concentrations of the compound were 9 concentration points obtained by a 5-fold serial dilution from 10 µM. A blank control containing 0.5% DMSO was set up. The plate was incubated for 72 h in a 5% CO₂ cell incubator at 37 °C. On the fifth day, the 96-well cell plate was removed from the incubator, and CellTiter-Glo^{®} Luminescent Cell Viability Assay (Promega, G7573) was added at 50 µL/well. After the plate was left to stand at room temperature for 10 min, the luminescence signal values were measured using a multifunctional microplate reader (PerkinElmer, EnVision2015). The IC₅₀ value for the inhibitory activity of the compound was calculated using Graphpad Prism software.

**Table 2. The inhibitory activity of the compound of the present disclosure against MIA PaCa-2 cell proliferation**

| Compound No. | IC₅₀ (nM) |
|---|---|
| 1 | 0.28 |

### Example 2: Preparation of Crystal Form A

5 mg of the compound represented by formula 1 was weighed, and 0.5 mL of ethyl acetate/n-heptane (v/v = 1:1) was added. The mixture was stirred at room temperature for 2 days and centrifuged, and the resulting solid was then dried *in vacuo* to give a solid. According to X-ray powder diffraction analysis, the product was defined as crystal form A. Its XRPD pattern is shown in FIG. 1, and its characteristic peak positions are shown in Table 3. Its DSC thermogram showed endothermic peaks at temperatures of 156.75 °C and 206.08 °C. Its TGA thermogram showed a weight loss of 10.64% from 30 °C to 140 °C.

**Table 3**

| Peak No. | 2θ [° or degrees] | d [Å] | Relative intensity (%) |
|---|---|---|---|
| 1 | 7.419 | 11.90635 | 16.2 |
| 2 | 8.215 | 10.75473 | 47.0 |
| 3 | 8.406 | 10.51010 | 75.0 |
| 4 | 9.986 | 8.85030 | 16.2 |
| 5 | 10.960 | 8.06635 | 91.7 |
| 6 | 11.530 | 7.66838 | 55.3 |
| 7 | 15.348 | 5.76847 | 32.3 |
| 8 | 15.430 | 5.73805 | 30.0 |
| 9 | 15.946 | 5.55335 | 19.4 |
| 10 | 16.362 | 5.41318 | 33.7 |
| 11 | 16.425 | 5.39256 | 30.0 |
| 12 | 16.773 | 5.28135 | 22.2 |
| 13 | 17.168 | 5.16092 | 11.2 |
| 14 | 17.495 | 5.06513 | 15.3 |
| 15 | 18.216 | 4.86614 | 100.0 |
| 16 | 20.437 | 4.34218 | 19.5 |
| 17 | 20.841 | 4.25876 | 50.2 |
| 18 | 21.025 | 4.22191 | 33.5 |
| 19 | 21.298 | 4.16845 | 48.9 |
| 20 | 21.667 | 4.09839 | 16.5 |
| 21 | 22.433 | 3.96011 | 33.1 |
| 22 | 22.849 | 3.88883 | 39.8 |
| 23 | 23.011 | 3.86189 | 44.2 |
| 24 | 24.090 | 3.69124 | 33.0 |
| 25 | 24.196 | 3.67538 | 23.9 |
| 26 | 25.125 | 3.54158 | 20.1 |
| 27 | 25.582 | 3.47926 | 15.6 |
| 28 | 26.211 | 3.39716 | 16.1 |
| 29 | 26.335 | 3.38155 | 17.6 |
| 30 | 26.867 | 3.31574 | 9.2 |
| 31 | 27.477 | 3.24348 | 7.4 |
| 32 | 27.830 | 3.20320 | 8.1 |
| 33 | 28.948 | 3.08191 | 33.6 |
| 34 | 29.801 | 2.99564 | 10.3 |
| 35 | 30.452 | 2.93302 | 18.1 |
| 36 | 31.392 | 2.84731 | 6.8 |
| 37 | 31.764 | 2.81486 | 11.9 |
| 38 | 32.403 | 2.76076 | 7.7 |
| 39 | 33.726 | 2.65544 | 8.9 |
| 40 | 34.415 | 2.60383 | 4.8 |
| 41 | 38.072 | 2.36171 | 6.5 |

### Example 3: Preparation of Crystal Form A

20 mg of the compound represented by formula 1 was weighed, and 0.5 mL of ethyl acetate was added. The mixture was heated and cooled between 50 °C and 5 °C (at a rate of ±0.75 °C/min) with stirring for 1 day, and centrifuged. The resulting solid was then dried *in vacuo* to give the title product. According to X-ray powder diffraction analysis, the product was crystal form A.

### Example 4: Preparation of Crystal Form C

10 mg of the compound represented by formula 1 was weighed, and 1 mL of isopropanol was added. The mixture was stirred at room temperature for 3 days and centrifuged, and drying was then performed *in vacuo* to give a solid. According to X-ray powder diffraction analysis, the product was defined as crystal form C. Its XRPD pattern is shown in FIG. 2, and its characteristic peak positions are shown in Table 4. Its DSC thermogram showed an endothermic peak at a temperature of 239.41 °C. Its TGA thermogram showed a weight loss of 0.50% from 30 °C to 159 °C and a weight loss of 5.50% from 159 °C to 269 °C.

**Table 4**

| Peak No. | 2θ [° or degrees] | d [Å] | Relative intensity (%) |
|---|---|---|---|
| 1 | 5.995 | 14.73075 | 7.0 |
| 2 | 9.150 | 9.65671 | 100.0 |
| 3 | 11.840 | 7.46855 | 16.6 |
| 4 | 12.034 | 7.34861 | 32.1 |
| 5 | 12.812 | 6.90404 | 5.0 |
| 6 | 16.151 | 5.48354 | 14.3 |
| 7 | 17.102 | 5.18053 | 14.5 |
| 8 | 17.426 | 5.08498 | 8.3 |
| 9 | 17.860 | 4.96246 | 99.7 |
| 10 | 18.335 | 4.83495 | 2.9 |
| 11 | 19.920 | 4.45364 | 86.3 |
| 12 | 20.476 | 4.33382 | 5.1 |
| 13 | 22.365 | 3.97196 | 5.4 |
| 14 | 23.604 | 3.76620 | 46.9 |
| 15 | 23.997 | 3.70536 | 50.6 |
| 16 | 25.182 | 3.53369 | 16.0 |
| 17 | 25.803 | 3.45003 | 19.3 |
| 18 | 27.536 | 3.23670 | 18.0 |
| 19 | 28.038 | 3.17988 | 14.7 |
| 20 | 28.382 | 3.14210 | 4.2 |
| 21 | 28.821 | 3.09520 | 6.2 |
| 22 | 30.743 | 2.90595 | 8.7 |
| 23 | 31.353 | 2.85076 | 9.6 |
| 24 | 31.857 | 2.80681 | 8.0 |
| 25 | 32.633 | 2.74181 | 3.2 |
| 26 | 33.042 | 2.70886 | 3.9 |
| 27 | 34.101 | 2.62712 | 11.2 |
| 28 | 34.599 | 2.59039 | 4.2 |
| 29 | 35.263 | 2.54313 | 4.3 |
| 30 | 36.460 | 2.46234 | 3.1 |
| 31 | 37.136 | 2.41908 | 4.8 |

### Example 5: Preparation of Crystal Form C

10 mg of the compound represented by formula 1 was weighed, and a solvent was added. The solvent is shown in Table 5 below. After dissolution-crystallization at room temperature, the mixture was stirred for 3 days and centrifuged. The resulting solid was then dried *in vacuo* to give the title product. According to X-ray powder diffraction analysis, the product was crystal form C.

**Table 5**

| Solvent | Crystal form |
|---|---|
| 0.1 mL of acetone | C |
| 0.1 mL of tetrahydrofuran | C |
| 0.1 mL of 1,4-dioxane | C |
| 0.4 mL of isopropyl acetate | C |
| 1.0 mL of dichloromethane | C |

### Example 6: Preparation of Crystal Form C

5 mg of the compound represented by formula 1 was weighed and dissolved in 0.05 mL of 1,4-dioxane by stirring at room temperature, and 0.45 mL of a solvent was added. The solvent is shown in Table 6 below. The mixture was stirred for crystallization, slurried at room temperature for 3 days, and centrifuged. The resulting solid was then dried *in vacuo* to give the title product. According to X-ray powder diffraction analysis, the product was crystal form C.

**Table 6**

| Solvent | Crystal form |
|---|---|
| Isopropanol | C |
| Methyl *tert*-butyl ether | C |
| *n*-Heptane | C |
| Isopropyl acetate | C |
| Dichloromethane | C |
| Cyclohexane | C |

### Example 7: Preparation of Crystal Form C

10 mg of the compound represented by formula 1 was weighed, and 0.2 mL of *n*-propanol was added. The mixture was slurried at 60 °C for 1 day and centrifuged. The resulting solid was then dried *in vacuo* to give the title product. According to X-ray powder diffraction analysis, the product was crystal form C.

### Example 8: Preparation of Crystal Form D

80 mg of the compound represented by formula 1 was weighed, and 1 mL of *n*-butanol was added. The mixture was heated and cooled between 50 °C and 5 °C (at a rate of ±0.75 °C/min) with stirring for 2 days, and centrifuged. The resulting solid was then dried *in vacuo* to give a solid. According to X-ray powder diffraction analysis, the product was defined as crystal form D. Its XRPD pattern is shown in FIG. 3, and its characteristic peak positions are shown in Table 7. Its DSC thermogram showed an endothermic peak at a temperature of 235.17 °C. Its TGA thermogram showed a weight loss of 1.10% from 30 °C to 119 °C and a weight loss of 5.62% from 120 °C to 266 °C.

**Table 7**

| Peak No. | 2θ [° or degrees] | d [Å] | Relative intensity (%) |
|---|---|---|---|
| 1 | 6.042 | 14.61693 | 9.4 |
| 2 | 9.096 | 9.71408 | 27.1 |
| 3 | 11.735 | 7.53531 | 41.6 |
| 4 | 12.094 | 7.31206 | 70.1 |
| 5 | 12.833 | 6.89251 | 4.0 |
| 6 | 14.894 | 5.94308 | 11.4 |
| 7 | 16.002 | 5.53409 | 9.9 |
| 8 | 16.610 | 5.33279 | 5.4 |
| 9 | 16.976 | 5.21886 | 9.8 |
| 10 | 17.222 | 5.14485 | 8.1 |
| 11 | 17.928 | 4.94365 | 100.0 |
| 12 | 19.870 | 4.46469 | 45.6 |
| 13 | 20.317 | 4.36757 | 23.9 |
| 14 | 21.905 | 4.05439 | 15.6 |
| 15 | 22.032 | 4.03122 | 10.0 |
| 16 | 22.870 | 3.88543 | 3.8 |
| 17 | 23.397 | 3.79897 | 26.0 |
| 18 | 23.968 | 3.70984 | 41.1 |
| 19 | 24.605 | 3.61524 | 9.7 |
| 20 | 24.917 | 3.57059 | 11.0 |
| 21 | 25.560 | 3.48229 | 5.9 |
| 22 | 26.212 | 3.39705 | 3.4 |
| 23 | 27.253 | 3.26968 | 11.9 |
| 24 | 27.473 | 3.24400 | 9.8 |
| 25 | 27.951 | 3.18953 | 5.7 |
| 26 | 28.396 | 3.14053 | 7.0 |
| 27 | 29.556 | 3.01986 | 8.6 |
| 28 | 30.874 | 2.89393 | 4.3 |
| 29 | 32.367 | 2.76379 | 3.3 |
| 30 | 33.991 | 2.63535 | 5.0 |
| 31 | 34.281 | 2.61373 | 4.9 |
| 32 | 34.913 | 2.56786 | 6.7 |
| 33 | 35.830 | 2.50417 | 4.4 |
| 34 | 36.583 | 2.45434 | 5.9 |
| 35 | 39.081 | 2.30305 | 3.7 |
| 36 | 39.392 | 2.28556 | 3.3 |
| 37 | 40.063 | 2.24881 | 4.3 |
| 38 | 40.448 | 2.22828 | 5.4 |
| 39 | 40.896 | 2.20488 | 3.8 |
| 40 | 44.358 | 2.04051 | 4.6 |

### Example 9: Preparation of Crystal Form D

10 mg of the compound represented by formula 1 was weighed, and 0.2 mL of *tert*-butanol was added. The mixture was slurried at 60 °C for 1 day and centrifuged. The resulting solid was then dried *in vacuo* to give a solid. According to X-ray powder diffraction analysis, the product was crystal form D.

### Example 10: Preparation of Crystal Form E

10 mg of the compound represented by formula 1 was weighed, and 1 mL of methanol was added. The mixture was heated to 50 °C for dissolution, followed by slow volatilization to give a solid. According to X-ray powder diffraction analysis, the product was defined as crystal form E. Its XRPD pattern is shown in FIG. 4, and its characteristic peak positions are shown in Table 8. Its DSC thermogram showed endothermic peaks at temperatures of 229.16 °C and 259.36 °C. Its TGA thermogram showed a weight loss of 2.17% from 30 °C to 177 °C and a weight loss of 5.94% from 177 °C to 260 °C.

**Table 8**

| Peak No. | 2θ [° or degrees] | d [Å] | Relative intensity (%) |
|---|---|---|---|
| 1 | 7.296 | 12.10588 | 49.4 |
| 2 | 10.626 | 8.31891 | 88.0 |
| 3 | 11.393 | 7.76066 | 36.9 |
| 4 | 12.209 | 7.24359 | 8.1 |
| 5 | 12.647 | 6.99389 | 25.2 |
| 6 | 14.530 | 6.09146 | 4.3 |
| 7 | 15.385 | 5.75485 | 5.6 |
| 8 | 16.030 | 5.52469 | 17.0 |
| 9 | 17.635 | 5.02520 | 22.1 |
| 10 | 17.905 | 4.95005 | 100.0 |
| 11 | 20.461 | 4.33707 | 12.7 |
| 12 | 20.706 | 4.28624 | 29.8 |
| 13 | 21.215 | 4.18465 | 25.0 |
| 14 | 21.483 | 4.13301 | 35.3 |
| 15 | 21.730 | 4.08664 | 23.9 |
| 16 | 21.818 | 4.07021 | 27.5 |
| 17 | 22.585 | 3.93381 | 9.5 |
| 18 | 23.131 | 3.84208 | 38.5 |
| 19 | 23.313 | 3.81261 | 13.3 |
| 20 | 23.652 | 3.75865 | 6.5 |
| 21 | 24.081 | 3.69265 | 20.4 |
| 22 | 24.131 | 3.68519 | 12.5 |
| 23 | 24.729 | 3.59739 | 36.1 |
| 24 | 25.377 | 3.50695 | 41.8 |
| 25 | 26.630 | 3.34467 | 4.2 |
| 26 | 26.919 | 3.30940 | 15.1 |
| 27 | 26.991 | 3.30081 | 14.1 |
| 28 | 27.267 | 3.26797 | 30.6 |
| 29 | 27.497 | 3.24118 | 4.4 |
| 30 | 27.554 | 3.23455 | 7.1 |
| 31 | 28.037 | 3.17997 | 3.2 |
| 32 | 29.502 | 3.02530 | 6.5 |
| 33 | 29.796 | 2.99611 | 7.0 |
| 34 | 30.159 | 2.96088 | 6.5 |
| 35 | 30.742 | 2.90601 | 6.2 |
| 36 | 30.907 | 2.89092 | 15.9 |
| 37 | 32.086 | 2.78734 | 13.5 |
| 38 | 32.484 | 2.75408 | 13.2 |
| 39 | 32.773 | 2.73047 | 4.0 |
| 40 | 33.353 | 2.68427 | 5.2 |
| 41 | 33.775 | 2.65171 | 5.1 |
| 42 | 34.188 | 2.62060 | 3.5 |
| 43 | 34.666 | 2.58552 | 4.2 |
| 44 | 37.067 | 2.42342 | 6.4 |

### Example 11: Preparation of Crystal Form E

5 mg of the compound represented by formula 1 was weighed, and 0.2 mL of methanol was added. The mixture was heated and cooled between 50 °C and 5 °C (at a rate of ±0.75 °C/min) with stirring for 1 day, and centrifuged. The resulting solid was then dried *in vacuo* to give the title product. According to X-ray powder diffraction analysis, the product was crystal form E.

### Example 12: Preparation of Crystal Form E

5 mg of the compound represented by formula 1 was weighed, and 0.125 mL of 10% water/methanol was added. The mixture was heated and cooled between 50 °C and 5 °C (at a rate of ±0.75 °C/min) with stirring for 1 day, and centrifuged. The resulting solid was then dried *in vacuo* to give the title product. According to X-ray powder diffraction analysis, the product was crystal form E.

### Example 13: Preparation of Crystal Form F

1 g of the compound represented by formula 1 was weighed, and 13 mL of purified water was added. The mixture was heated and cooled between 50 °C and 5 °C (at a rate of ±0.75 °C/min) with stirring for 4 days, and filtered under reduced pressure. The resulting solid was then dried *in vacuo* to give a solid. According to X-ray powder diffraction analysis, the product was defined as crystal form F. Its XRPD pattern is shown in FIG. 5, and its characteristic peak positions are shown in Table 9. Its DSC thermogram showed endothermic peaks at temperatures of 85.97 °C, 150.99 °C, and 222.57 °C. Its TGA thermogram showed a weight loss of 6.03% from 30 °C to 101 °C. DVS testing showed that the sample had a hygroscopic weight gain of about 6.35% under normal storage conditions (i.e., 25 °C and 60% RH), a hygroscopic weight gain of about 6.44% under accelerated experimental conditions (i.e., 70% RH), and a hygroscopic weight gain of about 6.65% under extreme conditions (90% RH). In the process of the humidity changing from 0% to 95% RH, the desorption process of the sample did not overlap with its adsorption process. After DVS testing, the crystal form was re-identified, and the results showed that the crystal form did not change.

**Table 9**

| Peak No. | 2θ [° or degrees] | d [Å] | Relative intensity (%) |
|---|---|---|---|
| 1 | 7.935 | 11.13240 | 21.7 |
| 2 | 9.947 | 8.88478 | 69.2 |
| 3 | 10.339 | 8.54922 | 41.5 |
| 4 | 11.651 | 7.58917 | 26.0 |
| 5 | 11.990 | 7.37543 | 23.7 |
| 6 | 14.672 | 6.03273 | 5.2 |
| 7 | 15.672 | 5.64997 | 65.4 |
| 8 | 16.642 | 5.32268 | 37.0 |
| 9 | 17.032 | 5.20160 | 19.0 |
| 10 | 17.702 | 5.00635 | 27.0 |
| 11 | 18.016 | 4.91974 | 8.9 |
| 12 | 18.740 | 4.73124 | 77.2 |
| 13 | 19.933 | 4.45085 | 60.7 |
| 14 | 20.712 | 4.28513 | 100.0 |
| 15 | 21.471 | 4.13529 | 40.7 |
| 16 | 22.605 | 3.93034 | 6.4 |
| 17 | 23.364 | 3.80435 | 35.8 |
| 18 | 23.910 | 3.71875 | 73.6 |
| 19 | 25.504 | 3.48977 | 70.1 |
| 20 | 26.139 | 3.40645 | 60.2 |
| 21 | 26.881 | 3.31405 | 34.3 |
| 22 | 27.660 | 3.22247 | 33.2 |
| 23 | 27.994 | 3.18478 | 14.4 |
| 24 | 28.351 | 3.14549 | 62.1 |
| 25 | 28.898 | 3.08717 | 33.7 |
| 26 | 29.522 | 3.02328 | 7.4 |
| 27 | 29.974 | 2.97877 | 18.3 |
| 28 | 30.275 | 2.94977 | 12.1 |
| 29 | 30.904 | 2.89115 | 31.2 |
| 30 | 31.147 | 2.86918 | 11.8 |
| 31 | 31.777 | 2.81375 | 14.8 |
| 32 | 32.613 | 2.74344 | 18.7 |
| 33 | 33.019 | 2.71063 | 8.5 |
| 34 | 33.544 | 2.66942 | 12.3 |
| 35 | 34.317 | 2.61103 | 13.1 |
| 36 | 35.075 | 2.55630 | 5.8 |
| 37 | 35.387 | 2.53450 | 8.8 |
| 38 | 35.827 | 2.50440 | 5.3 |
| 39 | 36.475 | 2.46137 | 8.6 |
| 40 | 36.855 | 2.43683 | 12.4 |
| 41 | 37.257 | 2.41146 | 7.6 |
| 42 | 38.097 | 2.36024 | 20.1 |

### Example 14: Preparation of Crystal Form F

100 mg of the compound represented by formula 1 was weighed and dissolved in 1 mL of 80% acetone/water by stirring at room temperature, and 3.8 mL of purified water was added. The mixture was stirred for crystallization, heated to 50 °C, stirred for another 3 days, and centrifuged, and blow-drying was then performed at 40 °C overnight to give a solid. According to X-ray powder diffraction analysis, the product was crystal form F.

### Example 15: Preparation of Crystal Form G

80 mg of the compound represented by formula 1 was weighed, and 1 mL of methanol was added. The mixture was heated and cooled between 50 °C and 5 °C (at a rate of ±0.75 °C/min) with stirring for 2 days, and centrifuged. The resulting solid was then dried *in vacuo* at 80 °C for 7.5 h to give a solid. According to X-ray powder diffraction analysis, the product was defined as crystal form G. Its XRPD pattern is shown in FIG. 6, and its characteristic peak positions are shown in Table 10. Its DSC thermogram showed an endothermic peak at a temperature of 237.35 °C. Its TGA thermogram showed a weight loss of 0.59% from 30 °C to 100 °C and a weight loss of 0.43% from 100 °C to 270 °C. DVS testing showed that the sample had a hygroscopic weight gain of about 1.04% under normal storage conditions (i.e., 25 °C and 60% RH), a hygroscopic weight gain of about 1.21% under accelerated experimental conditions (i.e., 70% RH), and a hygroscopic weight gain of about 1.60% under extreme conditions (90% RH). In the process of the humidity changing from 0% to 95% RH, the desorption process of the sample substantially overlapped with its adsorption process. In addition, after DVS testing, the crystal form was re-identified, and the results showed that the crystal form did not change.

**Table 10**

| Peak No. | 2θ [° or degrees] | d [Å] | Relative intensity (%) |
|---|---|---|---|
| 1 | 7.457 | 11.84545 | 16.6 |
| 2 | 10.701 | 8.26043 | 90.1 |
| 3 | 11.896 | 7.43324 | 49.6 |
| 4 | 12.669 | 6.98160 | 80.6 |
| 5 | 15.704 | 5.63861 | 3.6 |
| 6 | 16.315 | 5.42872 | 8.7 |
| 7 | 17.822 | 4.97297 | 100.0 |
| 8 | 18.384 | 4.82211 | 9.1 |
| 9 | 19.949 | 4.44719 | 5.0 |
| 10 | 20.532 | 4.32227 | 38.8 |
| 11 | 21.015 | 4.22399 | 16.4 |
| 12 | 21.378 | 4.15310 | 33.5 |
| 13 | 22.429 | 3.96080 | 29.4 |
| 14 | 23.032 | 3.85850 | 15.1 |
| 15 | 23.185 | 3.83334 | 14.0 |
| 16 | 23.751 | 3.74314 | 4.8 |
| 17 | 24.176 | 3.67840 | 9.8 |
| 18 | 24.516 | 3.62806 | 35.1 |
| 19 | 25.246 | 3.52480 | 42.5 |
| 20 | 27.288 | 3.26554 | 46.8 |
| 21 | 29.294 | 3.04633 | 8.9 |
| 22 | 29.778 | 2.99784 | 8.8 |
| 23 | 30.424 | 2.93571 | 8.8 |
| 24 | 31.598 | 2.82922 | 11.4 |
| 25 | 31.902 | 2.80301 | 5.5 |
| 26 | 32.274 | 2.77148 | 4.9 |
| 27 | 32.704 | 2.73601 | 18.3 |
| 28 | 33.514 | 2.67172 | 5.5 |
| 29 | 33.890 | 2.64295 | 3.8 |
| 30 | 34.415 | 2.60382 | 5.7 |
| 31 | 37.555 | 2.39301 | 4.8 |

### Example 16: Preparation of Crystal Form G

500 mg of the compound represented by formula 1 was weighed and dissolved in 2 mL of 80% acetone/water by stirring at room temperature, and 8 mL of methanol and 6 mL of purified water were added. The mixture was stirred for another 4 days and filtered, and drying was then performed *in vacuo* at 80 °C overnight to give a solid. According to X-ray powder diffraction analysis, the product was crystal form G.

### Example 17: Preparation of Crystal Form G

10 mg of the crystal form E of the compound represented by formula 1 was weighed, and 0.2 mL of a solvent was added. The solvent is shown in Table 11 below. The mixture was stirred at 60 °C for 1 day and centrifuged, and drying was then performed *in vacuo* at 80 °C overnight to give a solid. According to X-ray powder diffraction analysis, the product was crystal form G.

**Table 11**

| Solvent | Crystal form |
|---|---|
| Isopropyl ether | G |
| Methyl *tert*-butyl ether | G |
| Cyclohexane | G |
| Isopropyl acetate | G |

### Example 18: Preparation of Crystal Form H

25 mg of the crystal form E of the compound represented by formula 1 was weighed, and 1 mL of diethyl ether was added. The mixture was slurried at room temperature or 50 °C for 4 h and centrifuged, and drying was then performed *in vacuo* at 80 °C overnight to give a solid. According to X-ray powder diffraction analysis, the product was defined as crystal form H. Its XRPD pattern is shown in FIG. 7, and its characteristic peak positions are shown in Table 12. Its DSC thermogram showed endothermic peaks at temperatures of 46.77 °C and 221.95 °C. Its TGA thermogram showed a weight loss of 2.23% from 30 °C to 100 °C and a weight loss of 0.20% from 100 °C to 220 °C.

**Table 12**

| Peak No. | 2θ [° or degrees] | d [Å] | Relative intensity (%) |
|---|---|---|---|
| 1 | 7.961 | 11.09720 | 100.0 |
| 2 | 13.533 | 6.53782 | 23.4 |
| 3 | 14.817 | 5.97382 | 6.1 |
| 4 | 15.180 | 5.83196 | 23.5 |
| 5 | 15.879 | 5.57683 | 22.8 |
| 6 | 15.946 | 5.55333 | 14.0 |
| 7 | 17.277 | 5.12846 | 4.0 |
| 8 | 18.591 | 4.76897 | 3.2 |
| 9 | 20.246 | 4.38272 | 3.9 |
| 10 | 20.619 | 4.30416 | 11.8 |
| 11 | 20.902 | 4.24646 | 8.4 |
| 12 | 22.595 | 3.93205 | 8.6 |
| 13 | 22.822 | 3.89344 | 7.5 |
| 14 | 23.545 | 3.77543 | 3.7 |
| 15 | 25.444 | 3.49784 | 5.1 |
| 16 | 26.215 | 3.39669 | 4.8 |
| 17 | 27.216 | 3.27400 | 5.0 |
| 18 | 27.564 | 3.23343 | 4.6 |
| 19 | 29.305 | 3.04521 | 5.7 |
| 20 | 30.005 | 2.97575 | 4.1 |
| 21 | 30.671 | 2.91258 | 12.8 |
| 22 | 32.290 | 2.77018 | 3.3 |

### Example 19: Preparation of Crystal Form H

25 mg of the compound represented by formula 1 was weighed, and 1 mL of diethyl ether was added. The mixture was slurried at room temperature for 5 days and centrifuged, and drying was then performed *in vacuo* at 40 °C overnight to give a solid. According to X-ray powder diffraction analysis, the product was crystal form H.

### Example 20: Preparation of Crystal Form I

30 mg of the compound represented by formula 1 was weighed, and 0.6 mL of n-pentanol was added. The mixture was slurried at 60 °C for 1 day and centrifuged, and drying was then performed *in vacuo* at 40 °C overnight to give the title product. According to X-ray powder diffraction analysis, the product was defined as crystal form I. Its XRPD pattern is shown in FIG. 8, and its characteristic peak positions are shown in Table 13. Its DSC thermogram showed endothermic peaks at temperatures of 66.03 °C, 222.56 °C, and 245.19 °C. Its TGA thermogram showed a weight loss of 0.97% from 30 °C to 100 °C and a weight loss of 8.15% from 100 °C to 240 °C.

**Table 13**

| Peak No. | 2θ [° or degrees] | d [Å] | Relative intensity (%) |
|---|---|---|---|
| 1 | 6.181 | 14.28797 | 7.9 |
| 2 | 8.996 | 9.82188 | 40.3 |
| 3 | 11.447 | 7.72394 | 6.6 |
| 4 | 11.630 | 7.60294 | 16.1 |
| 5 | 12.286 | 7.19852 | 54.7 |
| 6 | 12.916 | 6.84852 | 7.1 |
| 7 | 14.826 | 5.97044 | 8.3 |
| 8 | 15.616 | 5.67008 | 12.5 |
| 9 | 16.690 | 5.30764 | 21.1 |
| 10 | 17.786 | 4.98274 | 24.0 |
| 11 | 18.112 | 4.89387 | 100.0 |
| 12 | 18.430 | 4.81028 | 11.4 |
| 13 | 19.719 | 4.49852 | 63.3 |
| 14 | 20.042 | 4.42673 | 11.5 |
| 15 | 20.620 | 4.30397 | 8.1 |
| 16 | 21.437 | 4.14173 | 5.3 |
| 17 | 21.685 | 4.09487 | 4.7 |
| 18 | 22.812 | 3.89519 | 24.6 |
| 19 | 23.592 | 3.76810 | 11.6 |
| 20 | 24.005 | 3.70412 | 45.8 |
| 21 | 24.590 | 3.61736 | 5.6 |
| 22 | 25.027 | 3.55514 | 7.0 |
| 23 | 25.619 | 3.47432 | 4.5 |
| 24 | 25.880 | 3.43995 | 7.3 |
| 25 | 26.757 | 3.32909 | 15.1 |
| 26 | 27.096 | 3.28826 | 4.1 |
| 27 | 27.698 | 3.21815 | 10.7 |
| 28 | 28.721 | 3.10573 | 5.8 |
| 29 | 31.034 | 2.87935 | 5.4 |
| 30 | 31.429 | 2.84410 | 4.5 |
| 31 | 31.721 | 2.81856 | 6.3 |
| 32 | 34.529 | 2.59548 | 8.2 |

### Example 21: Preparation of Crystal Form J

30 mg of the compound represented by formula 1 was weighed, and 0.6 mL of isopentanol was added. The mixture was slurried at 60 °C for 1 day and centrifuged, and drying was then performed *in vacuo* at 40 °C overnight to give a solid. According to X-ray powder diffraction analysis, the product was defined as crystal form J. Its XRPD pattern is shown in FIG. 9, and its characteristic peak positions are shown in Table 14. Its DSC thermogram showed endothermic peaks at temperatures of 230.96 °C and 242.37 °C. Its TGA thermogram showed a weight loss of 0.46% from 31 °C to 100 °C and a weight loss of 7.85% from 171 °C to 260 °C.

**Table 14**

| Peak No. | 2θ [° or degrees] | d [Å] | Relative intensity (%) |
|---|---|---|---|
| 1 | 6.024 | 14.65989 | 14.5 |
| 2 | 8.928 | 9.89641 | 62.5 |
| 3 | 11.494 | 7.69274 | 9.2 |
| 4 | 11.725 | 7.54153 | 23.6 |
| 5 | 12.055 | 7.33578 | 59.3 |
| 6 | 12.655 | 6.98946 | 9.5 |
| 7 | 13.268 | 6.66769 | 2.8 |
| 8 | 14.807 | 5.97784 | 7.7 |
| 9 | 15.762 | 5.61799 | 17.2 |
| 10 | 16.671 | 5.31339 | 21.0 |
| 11 | 17.074 | 5.18893 | 7.3 |
| 12 | 17.555 | 5.04778 | 25.6 |
| 13 | 17.726 | 4.99951 | 100.0 |
| 14 | 18.123 | 4.89103 | 9.2 |
| 15 | 19.533 | 4.54102 | 73.6 |
| 16 | 19.781 | 4.48455 | 6.7 |
| 17 | 20.251 | 4.38150 | 12.9 |
| 18 | 21.589 | 4.11295 | 2.9 |
| 19 | 21.753 | 4.08221 | 4.3 |
| 20 | 22.714 | 3.91163 | 4.1 |
| 21 | 22.991 | 3.86522 | 28.4 |
| 22 | 23.333 | 3.80931 | 10.5 |
| 23 | 23.657 | 3.75794 | 35.0 |
| 24 | 24.540 | 3.62461 | 7.4 |
| 25 | 24.864 | 3.57814 | 3.4 |
| 26 | 25.118 | 3.54252 | 3.7 |
| 27 | 25.816 | 3.44834 | 3.8 |
| 28 | 26.826 | 3.32074 | 15.8 |
| 29 | 27.446 | 3.24706 | 12.3 |
| 30 | 28.293 | 3.15180 | 3.0 |
| 31 | 29.131 | 3.06296 | 2.9 |
| 32 | 29.539 | 3.02158 | 2.9 |
| 33 | 30.530 | 2.92579 | 3.0 |
| 34 | 30.970 | 2.88517 | 1.7 |
| 35 | 31.315 | 2.85420 | 4.9 |
| 36 | 31.904 | 2.80284 | 2.1 |
| 37 | 33.189 | 2.69719 | 2.9 |
| 38 | 33.931 | 2.63983 | 4.7 |
| 39 | 34.536 | 2.59501 | 2.2 |

### Example 22: Preparation of Crystal Form K

10 mg of the compound represented by formula 1 was weighed, and 1 mL of 7% water/ethanol was added. The mixture was stirred at room temperature for 3 days and centrifuged, and drying was then performed *in vacuo* to give a solid. According to X-ray powder diffraction analysis, the product was defined as crystal form K. Its XRPD pattern is shown in FIG. 10, and its characteristic peak positions are shown in Table 15. Its DSC thermogram showed an endothermic peak at a temperature of 240.73 °C. Its TGA thermogram showed a weight loss of 1.42% from 30 °C to 108 °C and a weight loss of 3.32% from 108 °C to 272 °C.

**Table 15**

| Peak No. | 2θ [° or degrees] | d [Å] | Relative intensity (%) |
|---|---|---|---|
| 1 | 6.121 | 14.42868 | 15.5 |
| 2 | 9.306 | 9.49607 | 17.2 |
| 3 | 12.132 | 7.28917 | 100.0 |
| 4 | 16.845 | 5.25902 | 3.4 |
| 5 | 17.933 | 4.94237 | 60.8 |
| 6 | 18.171 | 4.87803 | 15.3 |
| 7 | 20.058 | 4.42327 | 21.0 |
| 8 | 20.725 | 4.28238 | 3.5 |
| 9 | 23.484 | 3.78510 | 3.1 |
| 10 | 23.897 | 3.72072 | 6.0 |
| 11 | 24.077 | 3.69329 | 15.8 |
| 12 | 25.011 | 3.55739 | 2.3 |
| 13 | 25.421 | 3.50096 | 2.9 |
| 14 | 25.917 | 3.43503 | 2.7 |
| 15 | 27.738 | 3.21351 | 4.0 |
| 16 | 28.444 | 3.13538 | 3.5 |
| 17 | 28.891 | 3.08787 | 2.8 |
| 18 | 29.861 | 2.98973 | 2.6 |
| 19 | 30.857 | 2.89545 | 3.5 |
| 20 | 31.705 | 2.81991 | 4.8 |
| 21 | 31.924 | 2.80108 | 3.9 |
| 22 | 34.192 | 2.62034 | 8.9 |
| 23 | 35.298 | 2.54069 | 3.3 |
| 24 | 36.395 | 2.46656 | 3.3 |
| 25 | 36.591 | 2.45386 | 4.7 |

### Example 23: Preparation of Crystal Form K

5 mg of the compound represented by formula 1 was weighed and dissolved in 0.050 mL of 1,4-dioxane by stirring at room temperature, and 0.450 mL of ethanol was added. The mixture was stirred for crystallization, slurried at room temperature for 3 days, and centrifuged, and drying was then performed *in vacuo* to give a solid. According to X-ray powder diffraction analysis, the product was crystal form K.

### Example 24: Preparation of Crystal Form K

5 mg of the compound represented by formula 1 was weighed and dissolved in 0.075 mL of a mixed solvent of ethyl acetate/ethanol (v/v = 1:1) by stirring at room temperature. The mixture was heated and cooled between 50 °C and 5 °C (at a rate of ±0.75 °C/min) with stirring for 1 day, and centrifuged, and drying was then performed *in vacuo* to give a solid. According to X-ray powder diffraction analysis, the product was crystal form K.

### Example 25: Preparation of Crystal Form L

10 mg of the crystal form F of the compound represented by formula 1 was weighed, and 0.5 mL of water was added. The mixture was stirred at 95 °C for 2 h and centrifuged, and drying was then performed *in vacuo* to give a solid. According to X-ray powder diffraction analysis, the product was defined as crystal form L. Its XRPD pattern is shown in FIG. 11, and its characteristic peak positions are shown in Table 16. Its DSC thermogram showed endothermic peaks at temperatures of 98.46 °C and 237.45 °C. Its TGA thermogram showed a weight loss of 2.6% from 30 °C to 270 °C.

**Table 16**

| Peak No. | 2θ [° or degrees] | d [Å] | Relative intensity (%) |
|---|---|---|---|
| 1 | 6.580 | 13.42226 | 13.8 |
| 2 | 10.645 | 8.30407 | 13.5 |
| 3 | 12.936 | 6.83822 | 3.5 |
| 4 | 14.011 | 6.31574 | 11.5 |
| 5 | 14.586 | 6.06811 | 11.6 |
| 6 | 16.316 | 5.42819 | 6.0 |
| 7 | 16.725 | 5.29658 | 100.0 |
| 8 | 17.050 | 5.19613 | 22.3 |
| 9 | 18.475 | 4.79850 | 4.9 |
| 10 | 19.513 | 4.54549 | 21.0 |
| 11 | 19.780 | 4.48480 | 31.2 |
| 12 | 20.585 | 4.31122 | 10.1 |
| 13 | 20.927 | 4.24154 | 9.6 |
| 14 | 21.276 | 4.17277 | 4.1 |
| 15 | 21.862 | 4.06213 | 13.2 |
| 16 | 22.650 | 3.92259 | 5.4 |
| 17 | 23.529 | 3.77800 | 11.3 |
| 18 | 23.813 | 3.73358 | 6.0 |
| 20 | 26.090 | 3.41274 | 7.9 |
| 21 | 26.362 | 3.37808 | 5.6 |
| 22 | 26.952 | 3.30547 | 10.4 |
| 23 | 27.317 | 3.26209 | 13.1 |
| 24 | 27.932 | 3.19164 | 14.5 |
| 25 | 28.428 | 3.13710 | 4.3 |
| 26 | 28.886 | 3.08844 | 5.8 |
| 27 | 29.283 | 3.04738 | 8.8 |
| 28 | 30.201 | 2.95688 | 5.5 |
| 29 | 30.911 | 2.89054 | 7.9 |
| 32 | 32.916 | 2.71893 | 5.9 |
| 35 | 35.227 | 2.54563 | 4.0 |

### Example 26: Preparation of Crystal Form M

10 mg of the compound represented by formula 1 was weighed, and 0.1 mL of isopropyl acetate was added. The mixture was stirred at room temperature for 2 days and centrifuged, and drying was then performed *in vacuo* to give a solid. According to X-ray powder diffraction analysis, the product was defined as crystal form M. Its XRPD pattern is shown in FIG. 12, and its characteristic peak positions are shown in Table 17. Its DSC thermogram showed an endothermic peak at a temperature of 254.20 °C. Its TGA thermogram showed a weight loss of 0.23% from 27 °C to 247 °C. DVS testing showed that the sample had a hygroscopic weight gain of about 0.48% under normal storage conditions (i.e., 25 °C and 60% RH), a hygroscopic weight gain of about 0.55% under accelerated experimental conditions (i.e., 70% RH), and a hygroscopic weight gain of about 0.73% under extreme conditions (90% RH). In the process of the humidity changing from 0% to 95% RH, the desorption process of the sample substantially overlapped with its adsorption process. In addition, after DVS testing, the crystal form was re-identified, and the results showed that the crystal form did not change.

**Table 17**

| Peak No. | 2θ [° or degrees] | d [Å] | Relative intensity (%) |
|---|---|---|---|
| 1 | 7.595 | 11.63128 | 25.2 |
| 2 | 8.313 | 10.62711 | 34.0 |
| 3 | 10.451 | 8.45790 | 21.4 |
| 4 | 11.772 | 7.51126 | 72.1 |
| 5 | 12.680 | 6.97556 | 46.5 |
| 6 | 13.359 | 6.62241 | 39.5 |
| 7 | 13.791 | 6.41623 | 66.5 |
| 8 | 13.977 | 6.33096 | 36.8 |
| 9 | 15.173 | 5.83454 | 100.0 |
| 10 | 16.626 | 5.32779 | 26.5 |
| 11 | 17.320 | 5.11599 | 44.6 |
| 12 | 17.797 | 4.97974 | 35.3 |
| 13 | 18.762 | 4.72576 | 70.7 |
| 14 | 19.506 | 4.54715 | 23.2 |
| 15 | 20.945 | 4.23789 | 52.4 |
| 16 | 22.085 | 4.02167 | 11.5 |
| 17 | 22.434 | 3.95989 | 16.0 |
| 18 | 22.812 | 3.89510 | 24.9 |
| 19 | 23.587 | 3.76879 | 30.4 |
| 20 | 24.433 | 3.64032 | 21.5 |
| 21 | 25.511 | 3.48883 | 74.6 |
| 22 | 25.849 | 3.44391 | 88.4 |
| 23 | 26.857 | 3.31689 | 45.0 |
| 24 | 27.801 | 3.20645 | 9.0 |
| 25 | 28.471 | 3.13251 | 5.8 |
| 26 | 29.053 | 3.07107 | 7.9 |
| 27 | 29.605 | 3.01497 | 30.0 |
| 28 | 30.545 | 2.92431 | 22.5 |
| 30 | 31.952 | 2.79867 | 6.7 |
| 32 | 33.100 | 2.70423 | 6.2 |
| 35 | 34.168 | 2.62209 | 7.5 |
| 36 | 34.979 | 2.56310 | 6.5 |
| 37 | 36.045 | 2.48973 | 7.0 |
| 38 | 37.982 | 2.36712 | 8.0 |

### Example 27: Preparation of Crystal Form M

100 mg of the compound represented by formula 1 was weighed and dissolved in 2.5 mL of isopropyl acetate by stirring at 60 °C. After the mixture was cooled to room temperature, 1 mL of *n*-heptane was added. The mixture was stirred at room temperature overnight and centrifuged, and drying was then performed *in vacuo* to give a solid. According to X-ray powder diffraction analysis, the product was crystal form M.

### Example 28: Preparation of Crystal Form N

10 mg of the compound represented by formula 1 was weighed, and 0.1 mL of water was added. The mixture was stirred at 95 °C for 3 days and centrifuged, and drying was then performed *in vacuo* to give a solid. According to X-ray powder diffraction analysis, the product was defined as crystal form N. Its XRPD pattern is shown in FIG. 13, and its characteristic peak positions are shown in Table 18. Its DSC thermogram showed endothermic peaks at temperatures of 59.31 °C and 268.68 °C. Its TGA thermogram showed a weight loss of 2.1% from 25 °C to 213 °C. DVS testing showed that the sample had a hygroscopic weight gain of about 3.00% under normal storage conditions (i.e., 25 °C and 60% RH), a hygroscopic weight gain of about 3.06% under accelerated experimental conditions (i.e., 70% RH), and a hygroscopic weight gain of about 3.18% under extreme conditions (90% RH). In the process of the humidity changing from 0% to 95% RH, the desorption process of the sample substantially overlapped with its adsorption process. In addition, after DVS testing, the crystal form was re-identified, and the results showed that the crystal form did not change.

**Table 18**

| Peak No. | 2θ [° or degrees] | d [Å] | Relative intensity (%) |
|---|---|---|---|
| 1 | 6.324 | 13.96430 | 39.7 |
| 2 | 11.009 | 8.03005 | 11.6 |
| 3 | 11.594 | 7.62666 | 17.1 |
| 4 | 12.664 | 6.98451 | 23.4 |
| 5 | 13.092 | 6.75686 | 46.7 |
| 6 | 13.626 | 6.49350 | 100.0 |
| 7 | 14.699 | 6.02183 | 42.4 |
| 8 | 15.566 | 5.68826 | 36.3 |
| 9 | 18.048 | 4.91109 | 11.9 |
| 10 | 18.311 | 4.84129 | 6.4 |
| 11 | 19.483 | 4.55240 | 42.5 |
| 12 | 20.517 | 4.32543 | 24.2 |
| 13 | 20.724 | 4.28256 | 50.7 |
| 14 | 21.486 | 4.13243 | 9.0 |
| 15 | 21.740 | 4.08476 | 12.2 |
| 16 | 22.429 | 3.96075 | 51.3 |
| 17 | 23.287 | 3.81674 | 39.4 |
| 18 | 23.557 | 3.77364 | 20.5 |
| 19 | 24.162 | 3.68044 | 14.2 |
| 20 | 25.042 | 3.55310 | 23.3 |
| 21 | 25.492 | 3.49131 | 4.7 |
| 23 | 26.840 | 3.31900 | 23.6 |
| 24 | 27.407 | 3.25157 | 44.7 |
| 25 | 28.910 | 3.08584 | 19.9 |
| 26 | 29.743 | 3.00130 | 6.0 |
| 27 | 30.152 | 2.96158 | 21.6 |
| 28 | 30.969 | 2.88528 | 6.3 |
| 29 | 31.419 | 2.84491 | 15.5 |
| 30 | 32.992 | 2.71277 | 14.0 |
| 31 | 33.557 | 2.66840 | 7.7 |
| 32 | 34.945 | 2.56553 | 8.5 |

### Example 29: Preparation of Crystal Form N

10 mg of the compound represented by formula 1 was weighed and dissolved in 0.5 mL of ethyl acetate, and 1 mL of n-heptane was added. The mixture was stirred at room temperature overnight. A solid was precipitated, and then the mixture was filtered. The solid was taken and added to water. The resulting mixture was stirred at 95 °C overnight and centrifuged, and drying was then performed *in vacuo* to give a solid. According to X-ray powder diffraction analysis, the product was crystal form N.

### Example 30: Preparation of Crystal Form N

100 mg of the compound represented by formula 1 was weighed and dissolved in 1.0 mL of a mixed solution of methyl isobutyl ketone/isopropyl acetate (v/v = 1:1), and 5 mg of a seed crystal of crystal form N was added. The mixture was stirred at room temperature overnight and centrifuged. The resulting solid was then dried *in vacuo* to give the title product.

### Example 31: Preparation of Crystal Form N

100 mg of the compound represented by formula 1 was weighed, and 1.0 mL of methyl isobutyl ketone was added. The mixture was stirred at room temperature overnight and centrifuged, and drying was then performed *in vacuo* to give a solid. According to X-ray powder diffraction analysis, the product was crystal form N.

### Example 32: Preparation of Crystal Form O

50 mg of the compound represented by formula 1 was weighed and dissolved in 0.5 mL of 2-butanone, and 3.5 mL of water was added. The mixture was stirred at 50 °C for 2 h and centrifuged. The supernatant was removed, and a solid was obtained. According to X-ray powder diffraction analysis, the product was defined as crystal form O. Its XRPD pattern is shown in FIG. 14, and its characteristic peak positions are shown in Table 19.

**Table 19**

| Peak No. | 2θ [° or degrees] | d [Å] | Relative intensity (%) |
|---|---|---|---|
| 1 | 7.224 | 12.22765 | 96.0 |
| 2 | 8.423 | 10.48885 | 81.8 |
| 3 | 8.965 | 9.85560 | 38.9 |
| 4 | 11.843 | 7.46655 | 51.9 |
| 5 | 16.529 | 5.35886 | 37.2 |
| 6 | 16.829 | 5.26404 | 100.0 |
| 7 | 17.985 | 4.92807 | 18.9 |
| 8 | 19.735 | 4.49488 | 48.6 |
| 9 | 20.425 | 4.34469 | 71.3 |
| 10 | 21.943 | 4.04745 | 15.1 |
| 11 | 22.446 | 3.95775 | 4.0 |
| 12 | 23.309 | 3.81314 | 22.6 |
| 13 | 24.637 | 3.61060 | 29.6 |
| 14 | 25.297 | 3.51786 | 40.8 |
| 15 | 26.296 | 3.38646 | 25.0 |
| 16 | 26.904 | 3.31124 | 13.8 |
| 17 | 28.609 | 3.11766 | 20.3 |

### Example 33: Preparation of Crystal Form P

50 mg of the compound represented by formula 1 was weighed and dissolved in 0.5 mL of 2-butanone, and 3.5 mL of water was added. The mixture was stirred at 50 °C for 24 h and centrifuged. The supernatant was removed, and a solid was obtained. According to X-ray powder diffraction analysis, the product was defined as free-state crystal form P. Its XRPD pattern is shown in FIG. 15, and its characteristic peak positions are shown in Table 20.

**Table 20**

| Peak No. | 2θ [° or degrees] | d [Å] | Relative intensity (%) |
|---|---|---|---|
| 1 | 7.848 | 11.25587 | 100.0 |
| 2 | 8.105 | 10.90001 | 15.7 |
| 3 | 11.266 | 7.84788 | 4.3 |
| 4 | 13.063 | 6.77167 | 12.7 |
| 5 | 14.625 | 6.05184 | 5.7 |
| 6 | 16.342 | 5.41964 | 15.6 |
| 7 | 17.230 | 5.14242 | 5.3 |
| 8 | 17.961 | 4.93471 | 2.5 |
| 9 | 18.479 | 4.79744 | 8.5 |
| 10 | 19.322 | 4.58997 | 11.5 |
| 11 | 19.613 | 4.52253 | 16.8 |
| 12 | 20.149 | 4.40354 | 6.2 |
| 13 | 21.305 | 4.16717 | 12.3 |
| 14 | 23.584 | 3.76928 | 6.1 |
| 15 | 24.786 | 3.58919 | 9.3 |
| 16 | 25.468 | 3.49462 | 5.5 |
| 17 | 26.013 | 3.42260 | 5.8 |
| 18 | 28.796 | 3.09787 | 3.9 |
| 20 | 30.323 | 2.94520 | 4.3 |

### Example 34: Preparation of Crystal Form Q

50 mg of the compound represented by formula 1 was weighed and dissolved in 0.5 mL of 2-butanone, and 3.5 mL of water was added. The mixture was stirred at 50 °C for 2 h and centrifuged. The resulting solid was taken and dried *in vacuo* to give the title product. According to X-ray powder diffraction analysis, the product was defined as free-state crystal form Q. Its XRPD pattern is shown in FIG. 16, and its characteristic peak positions are shown in Table 21.

**Table 21**

| Peak No. | 2θ [° or degrees] | d [Å] | Relative intensity (%) |
|---|---|---|---|
| 1 | 7.921 | 11.15222 | 43.3 |
| 2 | 8.455 | 10.44913 | 100.0 |
| 3 | 11.443 | 7.72655 | 12.2 |
| 4 | 14.285 | 6.19529 | 12.0 |
| 5 | 15.468 | 5.72383 | 33.9 |
| 6 | 18.035 | 4.91461 | 31.3 |
| 7 | 19.739 | 4.49397 | 13.0 |
| 8 | 20.330 | 4.36463 | 14.2 |
| 9 | 22.224 | 3.99679 | 19.8 |
| 10 | 25.107 | 3.54397 | 14.1 |
| 11 | 26.015 | 3.42238 | 16.2 |

### Example 35: Preparation of Crystal Form R

50 mg of the compound represented by formula 1 was weighed and dissolved in 0.5 mL of 2-butanone, and 3.5 mL of water was added. The mixture was stirred at 50 °C for 2 h and centrifuged. The resulting solid was taken and dried *in vacuo.* 1.0 mL of water was then added. The resulting mixture was stirred at 60 °C for 12 h and centrifuged, and drying was performed *in vacuo* to give a solid. According to X-ray powder diffraction analysis, the product was defined as crystal form R. Its XRPD pattern is shown in FIG. 17, and its characteristic peak positions are shown in Table 22.

**Table 22**

| Peak No. | 2θ [° or degrees] | d [Å] | Relative intensity (%) |
|---|---|---|---|
| 1 | 6.374 | 13.85604 | 22.7 |
| 2 | 8.248 | 10.71132 | 34.2 |
| 3 | 9.598 | 9.20703 | 49.7 |
| 4 | 9.884 | 8.94195 | 35.3 |
| 5 | 10.537 | 8.38915 | 22.3 |
| 6 | 11.283 | 7.83613 | 61.5 |
| 7 | 11.748 | 7.52673 | 23.8 |
| 8 | 12.828 | 6.89533 | 55.3 |
| 9 | 13.705 | 6.45627 | 100.0 |
| 10 | 14.549 | 6.08350 | 18.4 |
| 11 | 15.045 | 5.88375 | 62.4 |
| 12 | 15.539 | 5.69803 | 51.5 |
| 13 | 16.670 | 5.31388 | 25.9 |
| 14 | 17.686 | 5.01069 | 30.3 |
| 15 | 18.729 | 4.73417 | 23.0 |
| 16 | 19.385 | 4.57530 | 34.2 |
| 17 | 19.714 | 4.49968 | 54.7 |
| 18 | 20.328 | 4.36510 | 7.0 |
| 19 | 20.791 | 4.26899 | 28.8 |
| 20 | 21.955 | 4.04527 | 12.0 |
| 21 | 22.430 | 3.96062 | 49.9 |
| 22 | 22.679 | 3.91765 | 35.7 |
| 23 | 22.968 | 3.86904 | 30.3 |
| 24 | 24.020 | 3.70196 | 72.9 |
| 25 | 25.017 | 3.55656 | 25.0 |
| 27 | 26.248 | 3.39255 | 38.0 |
| 28 | 27.107 | 3.28695 | 22.2 |
| 30 | 28.454 | 3.13434 | 9.1 |
| 31 | 29.213 | 3.05458 | 13.3 |
| 32 | 29.736 | 3.00199 | 10.7 |
| 33 | 30.427 | 2.93545 | 10.9 |

### Example 36: Preparation of Crystal Form S

50 mg of the compound represented by formula 1 was weighed, and 0.5 mL of methyl isobutyl ketone was added. The mixture was stirred at room temperature overnight and centrifuged, and drying was then performed *in vacuo* at 100 °C overnight to give a solid. According to X-ray powder diffraction analysis, the product was defined as free-state crystal form S. Its XRPD pattern is shown in FIG. 18, and its characteristic peak positions are shown in Table 23. Its DSC thermogram showed an endothermic peak at a temperature of 280.11 °C. Its TGA thermogram showed a weight loss of 0.52% from 33 °C to 333 °C.

**Table 23**

| Peak No. | 2θ [° or degrees] | d [Å] | Relative intensity (%) |
|---|---|---|---|
| 1 | 6.465 | 13.66047 | 15.2 |
| 2 | 10.810 | 8.17794 | 15.9 |
| 3 | 11.872 | 7.44871 | 26.9 |
| 4 | 12.996 | 6.80661 | 37.4 |
| 5 | 13.597 | 6.50722 | 100.0 |
| 6 | 14.706 | 6.01894 | 73.3 |
| 7 | 15.563 | 5.68913 | 29.1 |
| 8 | 16.385 | 5.40577 | 7.7 |
| 9 | 18.374 | 4.82481 | 7.2 |
| 10 | 19.971 | 4.44233 | 38.2 |
| 11 | 20.300 | 4.37109 | 15.2 |
| 12 | 20.914 | 4.24412 | 27.7 |
| 13 | 21.810 | 4.07166 | 6.4 |
| 14 | 22.036 | 4.03055 | 19.2 |
| 15 | 22.751 | 3.90545 | 46.2 |
| 16 | 23.548 | 3.77505 | 9.7 |
| 17 | 23.881 | 3.72310 | 41.4 |
| 18 | 24.318 | 3.65715 | 3.4 |
| 19 | 25.414 | 3.50188 | 14.5 |
| 21 | 26.322 | 3.38309 | 22.9 |
| 22 | 27.331 | 3.26048 | 7.0 |
| 23 | 27.928 | 3.19211 | 8.6 |
| 24 | 28.244 | 3.15710 | 7.3 |
| 25 | 29.235 | 3.05228 | 13.7 |
| 28 | 31.375 | 2.84885 | 11.8 |
| 29 | 31.520 | 2.83605 | 5.7 |
| 30 | 32.963 | 2.71511 | 6.0 |

### Example 37: Preparation of Crystal Form T

10 mg of the compound represented by formula 1 was weighed and added to 1 mL of isopropanol. The solution system was stirred at room temperature until it became clear, and then filtered through a 0.22 µm filter membrane. The solution was subjected to slow evaporation at room temperature for crystallization to give a solid. According to X-ray powder diffraction analysis, the product was defined as crystal form T. Its XRPD pattern is shown in FIG. 19, and its characteristic peak positions are shown in Table 24.

**Table 24**

| Peak No. | 2θ [° or degrees] | d [Å] | Relative intensity (%) |
|---|---|---|---|
| 1 | 6.431 | 13.73215 | 100.0 |
| 2 | 9.082 | 9.72921 | 3.5 |
| 3 | 12.001 | 7.36868 | 0.5 |
| 4 | 12.385 | 7.14131 | 3.8 |
| 5 | 12.585 | 7.02804 | 2.6 |
| 6 | 12.919 | 6.84687 | 0.6 |
| 7 | 13.493 | 6.55702 | 0.1 |
| 8 | 14.046 | 6.30014 | 3.3 |
| 9 | 16.507 | 5.36586 | 2.6 |
| 10 | 17.799 | 4.97926 | 1.5 |
| 11 | 17.949 | 4.93804 | 1.2 |
| 12 | 19.457 | 4.55859 | 2.4 |
| 13 | 19.884 | 4.46170 | 14.2 |
| 14 | 20.100 | 4.41409 | 3.8 |
| 15 | 20.321 | 4.36666 | 2.9 |
| 16 | 21.207 | 4.18611 | 19.6 |
| 17 | 21.993 | 4.03836 | 13.4 |
| 18 | 22.383 | 3.96881 | 0.9 |
| 19 | 23.738 | 3.74522 | 0.7 |
| 20 | 24.138 | 3.68400 | 16.0 |
| 21 | 24.924 | 3.56965 | 10.9 |
| 22 | 25.111 | 3.54348 | 8.8 |
| 23 | 27.407 | 3.25161 | 0.7 |
| 24 | 27.581 | 3.23154 | 0.8 |
| 25 | 27.991 | 3.18506 | 1.6 |
| 26 | 28.208 | 3.16113 | 2.2 |
| 27 | 33.297 | 2.68865 | 2.2 |
| 28 | 37.753 | 2.38094 | 0.9 |

### Example 38: Preparation of Crystal Form U

20 mg of the compound represented by formula 1 was weighed and added to a mixed solution of 0.18 mL of methanol and 0.02 mL of water. The mixture was stirred for 24 h and filtered, and the resulting product was then dried at 50 °C for 16 h to give a solid. According to X-ray powder diffraction analysis, the product was defined as crystal form U. Its XRPD pattern is shown in FIG. 20, and its characteristic peak positions are shown in Table 25.

**Table 25**

| Peak No. | 2θ [° or degrees] | d [Å] | Relative intensity (%) |
|---|---|---|---|
| 1 | 6.843 | 12.90763 | 65.5 |
| 2 | 7.185 | 12.29405 | 100.0 |
| 3 | 8.193 | 10.78342 | 69.6 |
| 4 | 9.197 | 9.60841 | 1.5 |
| 5 | 9.800 | 9.01794 | 3.6 |
| 6 | 11.408 | 7.75015 | 3.0 |
| 7 | 13.870 | 6.37970 | 8.0 |
| 8 | 14.416 | 6.13935 | 16.2 |
| 9 | 15.275 | 5.79573 | 2.9 |
| 10 | 16.683 | 5.30959 | 4.2 |
| 11 | 18.102 | 4.89653 | 6.8 |
| 12 | 18.721 | 4.73596 | 5.9 |
| 13 | 19.302 | 4.59474 | 2.6 |
| 14 | 20.769 | 4.27344 | 7.7 |
| 15 | 21.159 | 4.19561 | 1.8 |
| 16 | 22.807 | 3.89594 | 1.4 |
| 17 | 25.376 | 3.50712 | 1.0 |
| 18 | 26.382 | 3.37558 | 0.9 |
| 19 | 30.302 | 2.94724 | 0.7 |
| 20 | 35.443 | 2.53065 | 0.8 |
| 21 | 44.368 | 2.04007 | 1.0 |

### Example 39: Preparation of Free-State Crystal Form B

10 mg of the crystal form E of the compound represented by formula 1 was weighed, and 0.5 mL of ethyl acetate was added. The mixture was stirred at room temperature for 2 days and centrifuged, and drying was then performed *in vacuo* to give a solid. According to X-ray powder diffraction analysis, the product was defined as free-state crystal form B. Its XRPD pattern is shown in FIG. 22, and its characteristic peak positions are shown in Table 26.

**Table 26**

| Peak No. | 2θ [° or degrees] | d [Å] | Relative intensity (%) |
|---|---|---|---|
| 1 | 7.545 | 11.70784 | 16.1 |
| 2 | 8.587 | 10.28860 | 100.0 |
| 3 | 10.089 | 8.76042 | 28.8 |
| 4 | 11.117 | 7.95224 | 25.2 |
| 5 | 11.875 | 7.44642 | 30.7 |
| 6 | 15.809 | 5.60109 | 11.6 |
| 7 | 16.601 | 5.33584 | 12.3 |
| 8 | 16.987 | 5.21539 | 27.4 |
| 9 | 17.056 | 5.19456 | 24.3 |
| 10 | 18.397 | 4.81875 | 13.7 |
| 11 | 18.515 | 4.78831 | 11.2 |
| 12 | 18.838 | 4.70691 | 10.2 |
| 13 | 19.285 | 4.59884 | 9.8 |
| 14 | 20.617 | 4.30465 | 5.6 |
| 15 | 21.287 | 4.17051 | 14.3 |
| 16 | 21.717 | 4.08904 | 29.5 |
| 17 | 22.527 | 3.94376 | 19.5 |
| 18 | 22.881 | 3.88355 | 12.7 |
| 19 | 23.200 | 3.83088 | 14.2 |
| 20 | 23.436 | 3.79274 | 32.3 |
| 21 | 24.384 | 3.64746 | 5.5 |
| 22 | 24.619 | 3.61313 | 5.9 |
| 23 | 25.543 | 3.48456 | 12.6 |
| 24 | 26.541 | 3.35571 | 12.6 |
| 25 | 28.472 | 3.13238 | 6.1 |
| 26 | 29.276 | 3.04812 | 16.4 |
| 27 | 30.437 | 2.93450 | 14.5 |
| 28 | 32.573 | 2.74678 | 4.8 |
| 29 | 36.720 | 2.44552 | 7.0 |

### Example 40: Preparation of Crystal Form α

10 mg of the crystal form N of the compound represented by formula 1 was weighed, and 0.2 mL of methanol was added. The mixture was stirred at room temperature for crystallization and centrifuged, and drying was then performed *in vacuo* to give a solid. According to X-ray powder diffraction analysis, the product was defined as free-state crystal form α. Its XRPD pattern is shown in FIG. 23, and its characteristic peak positions are shown in Table 27. Its DSC thermogram showed endothermic peaks at temperatures of 42.51 °C and 262.81 °C. Its TGA thermogram showed a weight loss of 3.43% from 30 °C to 316 °C.

**Table 27**

| Peak No. | 2θ [° or degrees] | d [Å] | Relative intensity (%) |
|---|---|---|---|
| 1 | 6.622 | 13.33691 | 100.0 |
| 2 | 9.944 | 8.88756 | 18.4 |
| 3 | 11.103 | 7.96253 | 59.4 |
| 4 | 13.332 | 6.63597 | 3.5 |
| 5 | 13.840 | 6.39321 | 6.0 |
| 6 | 15.927 | 5.56006 | 7.9 |
| 7 | 17.932 | 4.94267 | 25.5 |
| 8 | 20.112 | 4.41159 | 8.6 |
| 9 | 21.551 | 4.12008 | 3.7 |
| 10 | 22.143 | 4.01122 | 30.4 |
| 11 | 23.602 | 3.76646 | 4.9 |
| 12 | 25.120 | 3.54224 | 14.4 |
| 13 | 29.026 | 3.07385 | 5.2 |
| 14 | 30.154 | 2.96131 | 3.6 |

### Example 41: Preparation of Crystal Form α

10 mg of the crystal form N of the compound represented by formula 1 was weighed, and 0.2 mL of a solvent was added. The solvent is shown in Table 27 below. The mixture was stirred at room temperature for crystallization and centrifuged, and drying was then performed *in vacuo* to give a solid. According to X-ray powder diffraction analysis, the product was free-state crystal form α.

**Table 28**

| Solvent | Crystal form |
|---|---|
| 50% methanol/water | α |
| 7% water/ethanol | α |
| 50% acetonitrile/methanol | α |

### Example 42: Preparation of Crystal Form β

10 mg of the crystal form B of the compound represented by formula 1 was weighed, and 0.1 mL of propylene glycol methyl ether was added. The mixture was stirred at room temperature for crystallization and centrifuged, and drying was then performed *in vacuo* to give a solid. According to X-ray powder diffraction analysis, the product was defined as free-state crystal form β. Its XRPD pattern is shown in FIG. 24, and its characteristic peak positions are shown in Table 29. Its DSC thermogram showed endothermic peaks at temperatures of 157.49 °C and 221.33 °C. Its TGA thermogram showed a weight loss of 7.16% from 30 °C to 187 °C.

**Table 29**

| Peak No. | 2θ [° or degrees] | d [Å] | Relative intensity (%) |
|---|---|---|---|
| 1 | 7.683 | 11.49705 | 100.0 |
| 2 | 11.501 | 7.68764 | 44.3 |
| 3 | 12.400 | 7.13259 | 34.6 |
| 4 | 14.114 | 6.26993 | 20.1 |
| 5 | 15.099 | 5.86319 | 9.9 |
| 6 | 15.616 | 5.67021 | 45.3 |
| 7 | 16.527 | 5.35948 | 19.4 |
| 8 | 19.040 | 4.65740 | 51.8 |
| 9 | 19.592 | 4.52736 | 15.0 |
| 10 | 19.861 | 4.46667 | 15.8 |
| 11 | 21.013 | 4.22428 | 5.5 |
| 12 | 21.815 | 4.07079 | 19.3 |
| 13 | 22.242 | 3.99356 | 9.8 |
| 14 | 22.856 | 3.88776 | 75.2 |
| 15 | 24.200 | 3.67482 | 27.8 |
| 16 | 24.802 | 3.58696 | 42.7 |
| 17 | 26.348 | 3.37990 | 14.8 |
| 18 | 26.963 | 3.30418 | 14.1 |
| 19 | 28.023 | 3.18155 | 12.6 |
| 20 | 28.906 | 3.08632 | 11.2 |
| 21 | 29.420 | 3.03353 | 15.8 |
| 22 | 30.692 | 2.91064 | 4.1 |
| 23 | 31.500 | 2.83786 | 11.6 |
| 24 | 33.726 | 2.65540 | 11.3 |
| 25 | 35.883 | 2.50059 | 7.9 |

### Example 43: Influencing Factor Stability Study on Crystal Forms

Samples of the free-state crystal form C, crystal form F, crystal form G, crystal form H, crystal form L, crystal form M, and crystal form N were spread in open containers and left to stand under conditions of light exposure (4500 Lux), high temperature (40 °C and 60 °C), and high humidity (75% RH and 92.5% RH) for 30 days to study their stability.

**Table 30. The influencing factor stability study on crystal form C**

| Condition | Time (days) | Color and state | Purity (%) | Crystal form |
|---|---|---|---|---|
| Initial | 0 | Off-white solid | 98.2 | Crystal form C |
| Light exposure (4500 Lux) | 5 | Off-white solid | 98.0 | Did not change |
| | 10 | Off-white solid | 97.9 | Did not change |
| | 30 | Off-white solid | 97.5 | Did not change |
| 40 °C | 5 | Off-white solid | 98.0 | Did not change |
| | 10 | Off-white solid | 97.6 | Did not change |
| | 30 | Off-white solid | 97.2 | Did not change |
| 60 °C | 5 | Off-white solid | 98.0 | Did not change |
| | 10 | Off-white solid | 97.9 | Did not change |
| | 30 | Off-white solid | 97.5 | Did not change |
| 75% RH | 5 | Off-white solid | 98.1 | Did not change |
| | 10 | Off-white solid | 98.1 | Did not change |
| | 30 | Off-white solid | 98.1 | Did not change |
| 92.5% RH | 5 | Off-white solid | 98.1 | Did not change |
| | 10 | Off-white solid | 98.1 | Did not change |
| | 30 | Off-white solid | 98.1 | Did not change |

**Table 31. The influencing factor stability study on crystal form F**

| Condition | Time (days) | Color and state | Purity (%) | Crystal form |
|---|---|---|---|---|
| Initial | 0 | Off-white solid | 97.9 | Crystal form F |
| Light exposure | 5 | Off-white solid | 97.8 | Did not change |
| (4500 Lux) | 10 | Off-white solid | 97.8 | Did not change |
| | 30 | Off-white solid | 97.7 | Did not change |
| 40 °C | 5 | Off-white solid | 97.8 | Did not change |
| | 10 | Off-white solid | 97.5 | Did not change |
| | 30 | Off-white solid | 96.7 | Did not change |
| 60 °C | 5 | Off-white solid | 97.8 | Did not change |
| | 10 | Off-white solid | 97.7 | Did not change |
| | 30 | Off-white solid | 97.3 | Did not change |
| 75% RH | 5 | Off-white solid | 97.9 | Did not change |
| | 10 | Off-white solid | 97.9 | Did not change |
| | 30 | Off-white solid | 97.9 | Did not change |
| 92.5% RH | 5 | Off-white solid | 97.9 | Did not change |
| | 10 | Off-white solid | 97.9 | Did not change |
| | 30 | Off-white solid | 97.9 | Did not change |

**Table 32. The influencing factor stability study on crystal form G**

| Condition | Time (days) | Color and state | Purity (%) | Crystal form |
|---|---|---|---|---|
| Initial | 0 | Off-white solid | 98.5 | Crystal form G |
| Light exposure (4500 Lux) | 7 | Off-white solid | 98.5 | Did not change |
| | 14 | Off-white solid | 98.4 | Did not change |
| | 30 | Off-white solid | 98.3 | Did not change |
| 40 °C | 7 | Off-white solid | 98.2 | Did not change |
| | 14 | Off-white solid | 97.8 | Did not change |
| | 30 | Off-white solid | 97.7 | Did not change |
| 60 °C | 7 | Off-white solid | 98.3 | Did not change |
| | 14 | Off-white solid | 98.1 | Did not change |
| | 30 | Off-white solid | 98.1 | Did not change |
| 75% RH | 7 | Off-white solid | 98.5 | Did not change |
| | 14 | Off-white solid | 98.5 | Did not change |
| | 30 | Off-white solid | 98.5 | Did not change |
| 92.5% RH | 7 | Off-white solid | 98.5 | Did not change |
| | 14 | Off-white solid | 98.5 | Did not change |
| | 30 | Off-white solid | 98.5 | Did not change |

**Table 33. The influencing factor stability study on crystal form H**

| Condition | Time (days) | Color and state | Purity (%) | Crystal form |
|---|---|---|---|---|
| Initial | 0 | Off-white solid | 98.3 | Crystal form H |
| | 7 | Off-white solid | 97.8 | Did not change |
| Light exposure (4500 Lux) | 14 | Off-white solid | 96.8 | Did not change |
| | 30 | Off-white solid | 94.0 | Did not change |
| 40 °C | 7 | Off-white solid | 98.2 | Did not change |
| | 14 | Off-white solid | 98.1 | Did not change |
| | 30 | Off-white solid | 97.7 | Did not change |
| 60 °C | 7 | Off-white solid | 98.3 | Did not change |
| | 14 | Off-white solid | 98.2 | Did not change |
| | 30 | Off-white solid | 97.7 | Did not change |
| 75% RH | 7 | Off-white solid | 98.4 | Did not change |
| | 14 | Off-white solid | 98.3 | Did not change |
| | 30 | Off-white solid | 98.4 | Did not change |
| 92.5% RH | 7 | Off-white solid | 98.3 | Did not change |
| | 14 | Off-white solid | 98.3 | Did not change |
| | 30 | Off-white solid | 98.3 | Did not change |

**Table 34. The influencing factor stability study on crystal form L**

| Condition | Time (days) | Color and state | Purity (%) | Crystal form |
|---|---|---|---|---|
| Initial | 0 | Off-white solid | 99.1 | Crystal form L |
| Light exposure (4500 Lux) | 7 | Off-white solid | 98.6 | Did not change |
| | 14 | Off-white solid | 98.2 | Did not change |
| | 30 | Off-white solid | 98.3 | Did not change |
| 40 °C | 7 | Off-white solid | 99.1 | Did not change |
| | 14 | Off-white solid | 99.0 | Did not change |
| | 30 | Off-white solid | 98.9 | Did not change |
| 60 °C | 7 | Off-white solid | 99.1 | Did not change |
| | 14 | Off-white solid | 99.0 | Did not change |
| | 30 | Off-white solid | 98.9 | Did not change |
| 75% RH | 7 | Off-white solid | 99.1 | Did not change |
| | 14 | Off-white solid | 99.1 | Did not change |
| | 30 | Off-white solid | 99.1 | Did not change |
| 92.5% RH | 7 | Off-white solid | 99.1 | Did not change |
| | 14 | Off-white solid | 99.1 | Did not change |
| | 30 | Off-white solid | 99.1 | Did not change |

**Table 35. The influencing factor stability study on crystal form M**

| Condition | Time | Color and state | Purity | Crystal form |
|---|---|---|---|---|
| | (days) | | (%) | |
| Initial | 0 | Off-white solid | 99.2 | Crystal form M |
| Light exposure (4500 Lux) | 7 | Off-white solid | 99.2 | Did not change |
| | 14 | Off-white solid | 99.2 | Did not change |
| | 30 | Off-white solid | 99.0 | Did not change |
| 40 °C | 7 | Off-white solid | 99.1 | Did not change |
| | 14 | Off-white solid | 99.1 | Did not change |
| | 30 | Off-white solid | 98.9 | Did not change |
| 60 °C | 7 | Off-white solid | 99.1 | Did not change |
| | 14 | Off-white solid | 99.1 | Did not change |
| | 30 | Off-white solid | 99.0 | Did not change |
| 75% RH | 7 | Off-white solid | 99.2 | Did not change |
| | 14 | Off-white solid | 99.2 | Did not change |
| | 30 | Off-white solid | 99.2 | Did not change |
| 92.5% RH | 7 | Off-white solid | 99.2 | Did not change |
| | 14 | Off-white solid | 99.2 | Did not change |
| | 30 | Off-white solid | 99.2 | Did not change |

**Table 36. The influencing factor stability study on crystal form N**

| Condition | Time (days) | Color and state | Purity (%) | Crystal form |
|---|---|---|---|---|
| Initial | 0 | Off-white solid | 99.1 | Crystal form N |
| Light exposure (4500 Lux) | 7 | Off-white solid | 99.1 | Did not change |
| | 14 | Off-white solid | 99.1 | Did not change |
| | 30 | Off-white solid | 99.0 | Did not change |
| 40 °C | 7 | Off-white solid | 99.1 | Did not change |
| | 14 | Off-white solid | 98.9 | Did not change |
| | 30 | Off-white solid | 98.6 | Did not change |
| 60 °C | 7 | Off-white solid | 99.1 | Did not change |
| | 14 | Off-white solid | 99.1 | Did not change |
| | 30 | Off-white solid | 98.9 | Did not change |
| 75% RH | 7 | Off-white solid | 99.2 | Did not change |
| | 14 | Off-white solid | 99.1 | Did not change |
| | 30 | Off-white solid | 99.1 | Did not change |
| 92.5% RH | 7 | Off-white solid | 99.1 | Did not change |
| | 14 | Off-white solid | 99.1 | Did not change |
| | 30 | Off-white solid | 99.2 | Did not change |

| | | | | |
|---|---|---|---|---|
| Conclusion: The influencing factor experiments showed that: standing under conditions of light exposure, high temperature (40 °C and 60 °C), and high humidity (75% RH and 92.5% RH) for 30 days, the crystal form C, crystal form F, crystal form G, crystal form H, crystal form L, crystal form M, and crystal form N exhibited both good physical stability and good chemical stability. | | | | |

### Example 44: Long-Term Accelerated Stability Study on Free-State Crystal Forms

The crystal form C, crystal form F, crystal form G, crystal form H, crystal form L, crystal form M, and crystal form N were left to stand under conditions of 25 °C/60% RH and 40 °C/75% RH to study their stability.

**Table 37. The long-term accelerated stability study on crystal form C**

| Test conditions | Time (months) | Purity (%) | Crystal form |
|---|---|---|---|
| Initial | | 98.0 | Crystal form C |
| 25 °C/60% RH | 1 | 98.0 | Did not change |
| | 2 | 98.0 | Did not change |
| | 3 | 98.0 | Did not change |
| | 6 | 98.0 | Did not change |
| 40 °C/75% RH | 1 | 98.0 | Did not change |
| | 2 | 98.0 | Did not change |
| | 3 | 98.0 | Did not change |
| | 6 | 98.0 | Did not change |

**Table 38. The long-term accelerated stability study on crystal form F**

| Test conditions | Time (months) | Purity (%) | Crystal form |
|---|---|---|---|
| Initial | | 97.9 | Crystal form F |
| 25 °C/60% RH | 1 | 98.0 | Did not change |
| | 2 | 97.9 | Did not change |
| | 3 | 97.9 | Did not change |
| | 6 | 97.8 | Did not change |
| 40 °C/75% RH | 1 | 97.9 | Did not change |
| | 2 | 97.9 | Did not change |
| | 3 | 97.9 | Did not change |
| | 6 | 97.8 | Did not change |

**Table 39. The long-term accelerated stability study on crystal form G**

| Test conditions | Time (months) | Purity (%) | Crystal form |
|---|---|---|---|
| Initial | | 98.5 | Crystal form G |
| 25 °C/60% RH | 1 | 98.4 | Did not change |
| | 2 | 98.4 | Did not change |
| | 3 | 98.4 | Did not change |
| | 6 | 98.4 | Did not change |
| 40 °C/75% RH | 1 | 98.4 | Did not change |
| | 2 | 98.4 | Did not change |
| | 3 | 98.4 | Did not change |
| | 6 | 98.4 | Did not change |

**Table 40. The long-term accelerated stability study on crystal form H**

| Test conditions | Time (months) | Purity (%) | Crystal form |
|---|---|---|---|
| Initial | | 98.3 | Crystal form H |
| 25 °C/60% RH | 1 | 98.4 | Did not change |
| | 2 | 98.3 | Did not change |
| | 3 | 98.4 | Did not change |
| | 6 | 98.3 | Did not change |
| 40 °C/75% RH | 1 | 98.3 | Did not change |
| | 2 | 98.3 | Did not change |
| | 3 | 98.4 | Did not change |
| | 6 | 98.4 | Did not change |

**Table 41. The long-term accelerated stability study on crystal form L**

| Test conditions | Time (months) | Purity (%) | Crystal form |
|---|---|---|---|
| Initial | | 99.1 | Crystal form L |
| 25 °C/60% RH | 1 | 99.1 | Did not change |
| | 2 | 99.1 | Did not change |
| | 3 | 99.1 | Did not change |
| | 6 | 99.1 | Did not change |
| 40 °C/75% RH | 1 | 99.1 | Did not change |
| | 2 | 99.1 | Did not change |
| | 3 | 99.1 | Did not change |
| | 6 | 99.1 | Did not change |

**Table 42. The long-term accelerated stability study on crystal form M**

| Test conditions | Time (months) | Purity (%) | Crystal form |
|---|---|---|---|
| Initial | | 99.2 | Crystal form M |
| 25 °C/60% RH | 1 | 99.2 | Did not change |
| | 2 | 99.2 | Did not change |
| | 3 | 99.2 | Did not change |
| | 6 | 99.2 | Did not change |
| 40 °C75% RH | 1 | 99.2 | Did not change |
| | 2 | 99.2 | Did not change |
| | 3 | 99.2 | Did not change |
| | 6 | 99.2 | Did not change |

**Table 43. The long-term accelerated stability study on crystal form N**

| Test conditions | Time (months) | Purity (%) | Crystal form |
|---|---|---|---|
| Initial | | 99.1 | Crystal form N |
| 25 °C/60% RH | 1 | 99.1 | Did not change |
| | 2 | 99.2 | Did not change |
| | 3 | 99.1 | Did not change |
| | 6 | 99.2 | Did not change |
| 40 °C75% RH | 1 | 99.1 | Did not change |
| | 2 | 99.1 | Did not change |
| | 3 | 99.1 | Did not change |
| | 6 | 99.1 | Did not change |

| | | | |
|---|---|---|---|
| Conclusion: The long-term accelerated experiments showed that: standing under conditions of 25 °C/60% RH and 40 °C/75% RH for 6 months, the crystal form C, crystal form F, crystal form G, crystal form H, crystal form L, crystal form M, and crystal form N exhibited both good physical stability and good chemical stability. | | | |

## Claims

1. A crystal form C of the compound represented by formula 1, wherein an X-ray powder diffraction pattern expressed in terms of 2*θ* angles, which are diffraction angles, of the crystal form has characteristic peaks at 9.150, 12.034, 17.860, 19.920, 23.604, and 23.997, preferably at 9.150, 11.840, 12.034, 17.860, 19.920, 23.604, 23.997, 25.803, 27.536, and 28.038, and more preferably at 9.150, 11.840, 12.034, 17.860, 19.920, 23.604, 23.997, 25.803, 27.536, 28.038, 30.743, and 31.353,

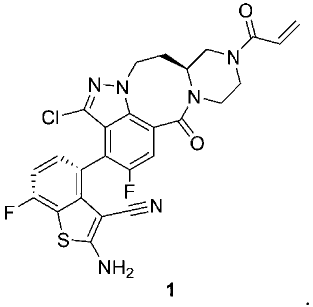

2. The crystal form C according to claim 1, wherein the X-ray powder diffraction pattern expressed in terms of 2*θ* angles, which are diffraction angles, of the crystal form is shown in FIG. 2.

3. A method for preparing the crystal form C according to claim 1 or 2, comprising:
method I: adding the compound of formula 1 to a solvent I, and stirring for crystallization, wherein the solvent I is selected from the group consisting of one or more of isopropanol, ethanol, and *n*-propanol;
method II: dissolving the compound of formula 1 in 1,4-dioxane, adding a solvent II, and stirring for crystallization, wherein the solvent II is selected from the group consisting of one or more of isopropanol, methyl *tert*-butyl ether, *n*-heptane, isopropyl acetate, dichloromethane, and cyclohexane;
method III: dissolving the compound of formula 1 in a solvent III, and stirring for crystallization, wherein the solvent III is selected from the group consisting of one or more of acetone, tetrahydrofuran, 1,4-dioxane, isopropyl acetate, dichloromethane, and tetrahydrofuran/ethanol (v/v = 2:1).

4. A crystal form F of the compound represented by formula 1, wherein an X-ray powder diffraction pattern expressed in terms of 2*θ* angles, which are diffraction angles, of the crystal form has characteristic peaks at 9.947, 15.672, 18.740, 20.712, 23.910, and 28.351, preferably at 7.935, 9.947, 10.339, 15.672, 18.740, 19.933, 20.712, 23.910, 25.504, 26.139, and 28.351, and more preferably at 7.935, 9.947, 10.339, 11.651, 11.990, 15.672, 16.642, 18.740, 19.933, 20.712, 21.471, 23.910, 25.504, 26.139, 27.660, 28.351, and 28.898.

5. The crystal form F according to claim 4, wherein the X-ray powder diffraction pattern expressed in terms of 2*θ* angles, which are diffraction angles, of the crystal form is shown in FIG. 5.

6. A method for preparing the crystal form F according to claim 4 or 5, comprising:
method I: adding the compound of formula 1 to purified water, and cyclically heating and cooling between 50 °C and 5 °C with stirring;
method II: dissolving the compound of formula 1 in 80% acetone/water, adding purified water, and stirring.

7. A crystal form G of the compound represented by formula 1, wherein an X-ray powder diffraction pattern expressed in terms of 2*θ* angles, which are diffraction angles, of the crystal form has characteristic peaks at 10.701, 11.896, 12.669, 17.822, 25.246, and 27.288, preferably at 7.457, 10.701, 11.896, 12.669, 17.822, 20.532, 21.378, 25.246, 27.288, and 32.704, and more preferably at 7.457, 10.701, 11.896, 12.669, 17.822, 20.532, 21.015, 21.378, 22.429, 24.516, 25.246, 27.288, and 32.704.

8. The crystal form G according to claim 7, wherein the X-ray powder diffraction pattern expressed in terms of 2*θ* angles, which are diffraction angles, of the crystal form is shown in FIG. 6.

9. A method for preparing the crystal form G according to claim 7 or 8, comprising:
method I: adding the compound of formula 1 to methanol, and cyclically heating and cooling between 50 °C and 5 °C with stirring;
method II: dissolving the compound of formula 1 in 80% acetone/water, adding methanol/water (V/V = 4:3), and stirring;
method III: adding a crystal form E of the compound of formula 1 to a solvent IV, and stirring, wherein the solvent IV is selected from the group consisting of one or more of isopropyl ether, methyl *tert*-butyl ether, cyclohexane, isopropyl acetate, and water.

10. A crystal form H of the compound represented by formula 1, wherein an X-ray powder diffraction pattern expressed in terms of 2*θ* angles, which are diffraction angles, of the crystal form has characteristic peaks at 7.961, 13.533, 15.180, 15.879, and 20.619, preferably at 7.961, 13.533, 15.180, 15.879, 15.946, 20.619, 20.902, 22.595, and 25.444, and more preferably at 7.961, 13.533, 15.180, 15.879, 15.946, 20.619, 20.902, 22.595, 25.444, 29.305, and 30.671.

11. The crystal form H according to claim 10, wherein the X-ray powder diffraction pattern expressed in terms of 2*θ* angles, which are diffraction angles, of the crystal form is shown in FIG. 7.

12. A method for preparing the crystal form H according to claim 10 or 11, comprising steps of adding the compound of formula 1 to diethyl ether and stirring.

13. A crystal form L of the compound represented by formula 1, wherein an X-ray powder diffraction pattern expressed in terms of 2*θ* angles, which are diffraction angles, of the crystal form has characteristic peaks at 10.645, 16.725, 17.050, 19.513, and 19.780, preferably at 6.580, 10.645, 14.011, 14.586, 16.725, 17.050, 19.513, 19.780, and 21.862, and more preferably at 6.580, 10.645, 14.011, 14.586, 16.725, 17.050, 19.513, 19.780, 21.862, 23.529, 26.952, and 27.932.

14. The crystal form L according to claim 13, wherein the X-ray powder diffraction pattern expressed in terms of 2*θ* angles, which are diffraction angles, of the crystal form is shown in FIG. 11.

15. A method for preparing the crystal form L according to claim 13 or 14, comprising steps of adding the crystal form F of the compound of formula 1 to water and stirring at 95 °C.

16. A crystal form M of the compound represented by formula 1, wherein an X-ray powder diffraction pattern expressed in terms of 2*θ* angles, which are diffraction angles, of the crystal form has characteristic peaks at 8.313, 11.772, 15.173, 18.762, 20.945, and 25.849, preferably at 7.595, 8.313, 11.772, 12.680, 13.791, 15.173, 17.320, 18.762, 20.945, 25.849, and 26.857, and more preferably at 7.595, 8.313, 11.772, 12.680, 13.359, 13.791, 15.173, 16.626, 17.320, 18.762, 19.506, 20.945, 25.849, 26.857, 29.605, and 30.545.

17. The crystal form M according to claim 16, wherein the X-ray powder diffraction pattern expressed in terms of 2*θ* angles, which are diffraction angles, of the crystal form is shown in FIG. 12.

18. A method for preparing the crystal form M according to claim 16 or 17, comprising:
method I: adding the compound of formula 1 to isopropyl acetate, and stirring;
method II: dissolving the compound of formula 1 in isopropyl acetate, adding *n*-heptane, and stirring.

19. A crystal form N of the compound represented by formula 1, wherein an X-ray powder diffraction pattern expressed in terms of 2*θ* angles, which are diffraction angles, of the crystal form has characteristic peaks at 6.324, 13.092, 13.626, 14.699, 19.483, 22.429, and 27.407, preferably at 6.324, 13.092, 13.626, 14.699, 15.566, 19.483, 20.724, 22.429, 23.287, and 27.407, and more preferably at 6.324, 12.664, 13.092, 13.626, 14.699, 15.566, 19.483, 20.724, 22.429, 23.287, 25.042, 26.840, 27.407, and 30.152.

20. The crystal form N according to claim 19, wherein the X-ray powder diffraction pattern expressed in terms of 2*θ* angles, which are diffraction angles, of the crystal form is shown in FIG. 13.

21. A method for preparing the crystal form N according to claim 19 or 20, comprising:
method I: adding the compound of formula 1 to a solvent V, and stirring for crystallization, wherein the solvent V is selected from the group consisting of water and methyl isobutyl ketone;
method II: dissolving the compound of formula 1 in a solvent VI, and adding a seed crystal for crystallization, wherein the solvent VI is selected from the group consisting of one or more of methyl isobutyl ketone and isopropyl acetate;
method III: dissolving the compound of formula 1 in ethyl acetate, adding n-heptane, and stirring.

22. A crystal form R of the compound represented by formula 1, wherein an X-ray powder diffraction pattern expressed in terms of 2*θ* angles, which are diffraction angles, of the crystal form has characteristic peaks at 9.598, 11.283, 13.705, 15.045, 19.714, and 24.020, preferably at 8.248, 9.598, 11.283, 12.828, 13.705, 15.045, 15.539, 19.714, 22.430, 24.020, and 26.248, and more preferably at 8.248, 9.598, 9.884, 11.283, 11.748, 12.828, 13.705, 15.045, 15.539, 16.670, 17.686, 19.714, 20.791, 22.430, 24.020, 25.017, and 26.248.

23. The crystal form R according to claim 22, wherein the X-ray powder diffraction pattern expressed in terms of 2*θ* angles, which are diffraction angles, of the crystal form is shown in FIG. 17.

24. A method for preparing the crystal form R according to claim 22 or 23, comprising:
step I: dissolving the compound of formula 1 in 2-butanone, adding water, and stirring for crystallization; and
step II: adding the crystal obtained in step I to water, and stirring at 60 °C for crystallization.

25. A crystal form S of the compound represented by formula 1, wherein an X-ray powder diffraction pattern expressed in terms of 2*θ* angles, which are diffraction angles, of the crystal form has characteristic peaks at 10.810, 13.597, 14.706, 19.971, and 22.751, preferably at 6.465, 10.810, 11.872, 13.597, 14.706, 15.563, 19.971, 22.751, 23.881, and 26.322, and more preferably at 6.465, 10.810, 11.872, 12.996, 13.597, 14.706, 15.563, 16.385, 19.971, 20.914, 22.751, 23.881, 25.414, 26.322, 29.235, and 32.963.

26. The crystal form S according to claim 25, wherein the X-ray powder diffraction pattern expressed in terms of 2*θ* angles, which are diffraction angles, of the crystal form is shown in FIG. 18.

27. A method for preparing the crystal form S according to claim 25 or 26, comprising:
step I: adding the compound of formula 1 to methyl isobutyl ketone, and stirring for crystallization; and
step II: drying the crystal obtained in step I *in vacuo* at 100 °C.

28. The crystal form according to any one of claims 1-2, 4-5, 7-8, 10-11, 13-14, 16-17, 19-20, 22-23, and 25-26, wherein the 2*θ* angles have a margin of error of ±0.20.

29. A pharmaceutical composition, comprising the crystal form according to any one of claims 1-2, 4-5, 7-8, 10-11, 13-14, 16-17, 19-20, 22-23, and 25-26, and optionally a pharmaceutically acceptable excipient.

30. A method for preparing a pharmaceutical composition, comprising a step of mixing the crystal form according to any one of claims 1-2, 4-5, 7-8, 10-11, 13-14, 16-17, 19-20, 22-23, and 25-26 with a pharmaceutically acceptable excipient.

31. Use of the crystal form according to any one of claims 1-2, 4-5, 7-8, 10-11, 13-14, 16-17, 19-20, 22-23, and 25-26, or the pharmaceutical composition according to claim 26 in the manufacture of a medicament for treating and/or preventing a tumor.
